# EUROPEAN PATENT SPECIFICATION

(11) **EP 2 662 386 B1**
(45) Date of publication and mention of the grant of the patent: **06.03.2019**
(21) Application number: 12003552.2
(22) Date of filing: 07.05.2012
(51) Int. Cl.: C07K 16/18, A61K 39/00

(54) **Antibodies that bind to PLAC1 and block interaction between PLAC1 and FGF7**
Antikörper, die an PLAC1 binden und die Wechselwirkung zwischen PLAC1 und FGF7 inhibieren
Anticorps qui se lient à PLAC1 et inhibent l'interaction entre PLAC1 et FGF7

(43) Date of publication of application: 13.11.2013
(73) Proprietor: Astellas Pharma Inc., Chuo-ku Tokyo 103-8411 (JP); TRON - Translationale Onkologie an der Universitätsmedizin der Johannes Gutenberg- Universität Mainz gemeinnützige GmbH, 55131 Mainz (DE); Universitätsmedizin der Johannes Gutenberg-Universität Mainz, 55131 Mainz (DE)
(72) Inventor: Sahin, Ugur, 55116 Mainz (DE); Koslowski, Michael, 65933 Frankfurt (DE); Grimmler, Matthias, 65604 Elz (DE); Barea Roldàn, Diana, 63069 Offenbach (DE); Hartmann, Christoph, 60316 Frankfurt (DE); Hubich, Stefanie, 55118 Mainz (DE)
(74) Representative: Schnappauf, Georg

(56) References cited:
- WO-A1-2012/007981
- MASSABBAL ELTAYAB ET AL: "PLAC1 expression increases during trophoblast differentiation: evidence for regulatory interactions with the fibroblast growth factor-7 (FGF-7) axis.", MOLECULAR REPRODUCTION AND DEVELOPMENT JUL 2005 LNKD- PUBMED:15803460, vol. 71, no. 3, July 2005 (2005-07), pages 299-304, XP002683563, ISSN: 1040-452X
- MICHAEL FANT ET AL: "PLAC1 (Placenta-specific 1): a novel, X-linked gene with roles in reproductive and cancer biology", PRENATAL DIAGNOSIS, 1 January 2010 (2010-01-01), pages n/a-n/a, XP55013261, ISSN: 0197-3851, DOI: 10.1002/pd.2506
- MICHAEL FANT ET AL: "The PLAC1 protein localizes to membranous compartments in the apical region of the syncytiotrophoblast", MOLECULAR REPRODUCTION AND DEVELOPMENT, vol. 74, no. 7, 1 July 2007 (2007-07-01), pages 922-929, XP55013187, ISSN: 1040-452X, DOI: 10.1002/mrd.20673
- KOSLOWSKI MICHAEL ET AL: "A placenta-specific gene ectopically activated in many human cancers is essentially involved in malignant cell processes", CANCER RESEARCH, AMERICAN ASSOCIATION FOR CANCER RESEARCH, US, vol. 67, no. 19, 1 October 2007 (2007-10-01), pages 9528-9534, XP002471063, ISSN: 0008-5472, DOI: 10.1158/0008-5472.CAN-07-1350
- SILVA W A ET AL: "PLAC1, a trophoblast-specific cell surface protein, is expressed in a range of human tumors and elicits spontaneous antibody responses", CANCER IMMUNITY, ACADEMY OF CANCER IMMUNOLOGY, CH, vol. 7, 6 November 2007 (2007-11-06), pages 1-9, XP002615924, ISSN: 1424-9634
- DONG XUE-YUAN ET AL: "PLAC1 is a tumor-specific antigen capable of eliciting spontaneous antibody responses in human cancer patients", INTERNATIONAL JOURNAL OF CANCER, JOHN WILEY & SONS, INC, NEW YORK, NY; US, vol. 122, no. 9, 1 May 2008 (2008-05-01), pages 2038-2043, XP009102971, ISSN: 0020-7136, DOI: 10.1002/IJC.23341

### SEQUENCE LISTING

<110> BioNTech AG et al.
<120> ANTIBODIES AND ANTIBODY MIXTURES FOR TREATMENT OF CANCER
<130> 674-72
<160> 3
<170> PatentIn version 3.5
<210> 1
   <211> 212
   <212> PRT
   <213> Homo sapiens
<400> 1
   Met Lys Val Phe Lys Phe Ile Gly Leu Met Ile Leu Leu Thr Ser Ala
   1               5                   10                  15
   Phe Ser Ala Gly Ser Gly Gln Ser Pro Met Thr Val Leu Cys Ser Ile
               20                  25                  30
   Asp Trp Phe Met Val Thr Val His Pro Phe Met Leu Asn Asn Asp Val
           35                  40                  45
   Cys Val His Phe His Glu Leu His Leu Gly Leu Gly Cys Pro Pro Asn
       50                  55                  60
   His Val Gln Pro His Ala Tyr Gln Phe Thr Tyr Arg Val Thr Glu Cys
   65                  70                  75                  80
   Gly Ile Arg Ala Lys Ala Val Ser Gln Asp Met Val Ile Tyr Ser Thr
                   85                  90                  95
   Glu Ile His Tyr Ser Ser Lys Gly Thr Pro Ser Lys Phe Val Ile Pro
               100                 105                 110
   Val Ser Cys Ala Ala Pro Gln Lys Ser Pro Trp Leu Thr Lys Pro Cys
           115                 120                 125
   Ser Met Arg Val Ala Ser Lys Ser Arg Ala Thr Ala Gln Lys Asp Glu
       130                 135                 140
   Lys Cys Tyr Glu Val Phe Ser Leu Ser Gln Ser Ser Gln Arg Pro Asn
   145                 150                 155                 160
   Cys Asp Cys Pro Pro Cys Val Phe Ser Glu Glu Glu His Thr Gln Val
                   165                 170                 175
   Pro Cys His Gln Ala Gly Ala Gln Glu Ala Gln Pro Leu Gln Pro Ser
               180                 185                 190
   His Phe Leu Asp Ile Ser Glu Asp Trp Ser Leu His Thr Asp Asp Met
   195 200 205
   Ile Gly Ser Met
       210
<210> 2
   <211> 194
   <212> PRT
   <213> Homo sapiens
<400> 2
   Met His Lys Trp Ile Leu Thr Trp Ile Leu Pro Thr Leu Leu Tyr Arg
   1 5 10 15
   Ser Cys Phe His Ile Ile Cys Leu Val Gly Thr Ile Ser Leu Ala Cys
   20 25 30
   Asn Asp Met Thr Pro Glu Gln Met Ala Thr Asn Val Asn Cys Ser Ser
   35 40 45
   Pro Glu Arg His Thr Arg Ser Tyr Asp Tyr Met Glu Gly Gly Asp Ile
   50 55 60
   Arg Val Arg Arg Leu Phe Cys Arg Thr Gln Trp Tyr Leu Arg Ile Asp
   65 70 75 80
   Lys Arg Gly Lys Val Lys Gly Thr Gln Glu Met Lys Asn Asn Tyr Asn
   85 90 95
   Ile Met Glu Ile Arg Thr Val Ala Val Gly Ile Val Ala Ile Lys Gly
   100 105 110
   Val Glu Ser Glu Phe Tyr Leu Ala Met Asn Lys Glu Gly Lys Leu Tyr
   115 120 125
   Ala Lys Lys Glu Cys Asn Glu Asp Cys Asn Phe Lys Glu Leu Ile Leu
   130 135 140
   Glu Asn His Tyr Asn Thr Tyr Ala Ser Ala Lys Trp Thr His Asn Gly
   145 150 155 160
   Gly Glu Met Phe Val Ala Leu Asn Gln Lys Gly Ile Pro Val Arg Gly
   165 170 175
   Lys Lys Thr Lys Lys Glu Gln Lys Thr Ala His Phe Leu Pro Met Ala
   180 185 190
   Ile Thr
<210> 3
   <211> 822
   <212> PRT
   <213> Homo sapiens
<400> 3
   Met Val Ser Trp Gly Arg Phe Ile Cys Leu Val Val Val Thr Met Ala
   1               5                   10                  15
   Thr Leu Ser Leu Ala Arg Pro Ser Phe Ser Leu Val Glu Asp Thr Thr
               20                  25                  30
   Leu Glu Pro Glu Glu Pro Pro Thr Lys Tyr Gln Ile Ser Gln Pro Glu
           35                  40                  45
   Val Tyr Val Ala Ala Pro Gly Glu Ser Leu Glu Val Arg Cys Leu Leu
       50                  55                  60
   Lys Asp Ala Ala Val Ile Ser Trp Thr Lys Asp Gly Val His Leu Gly
   65                  70                  75                  80
   Pro Asn Asn Arg Thr Val Leu Ile Gly Glu Tyr Leu Gln Ile Lys Gly
                   85                  90                  95
   Ala Thr Pro Arg Asp Ser Gly Leu Tyr Ala Cys Thr Ala Ser Arg Thr
               100                 105                 110
   Val Asp Ser Glu Thr Trp Tyr Phe Met Val Asn Val Thr Asp Ala Ile
           115                 120                 125
   Ser Ser Gly Asp Asp Glu Asp Asp Thr Asp Gly Ala Glu Asp Phe Val
       130                 135                 140
   Ser Glu Asn Ser Asn Asn Lys Arg Ala Pro Tyr Trp Thr Asn Thr Glu
   145                 150                 155                 160
   Lys Met Glu Lys Arg Leu His Ala Val Pro Ala Ala Asn Thr Val Lys
                   165                 170                 175
   Phe Arg Cys Pro Ala Gly Gly Asn Pro Met Pro Thr Met Arg Trp Leu
               180                 185                 190
   Lys Asn Gly Lys Glu Phe Lys Gln Glu His Arg Ile Gly Gly Tyr Lys
           195                 200                 205
   Val Arg Asn Gln His Trp Ser Leu Ile Met Glu Ser Val Val Pro Ser
       210                 215                 220
   Asp Lys Gly Asn Tyr Thr Cys Val Val Glu Asn Glu Tyr Gly Ser Ile
   225                 230                 235                 240
   Asn His Thr Tyr His Leu Asp Val Val Glu Arg Ser Pro His Arg Pro
                   245                 250                 255
   Ile Leu Gln Ala Gly Leu Pro Ala Asn Ala Ser Thr Val Val Gly Gly
               260                 265                 270
   Asp Val Glu Phe Val Cys Lys Val Tyr Ser Asp Ala Gln Pro His Ile
           275                 280                 285
   Gln Trp Ile Lys His Val Glu Lys Asn Gly Ser Lys Tyr Gly Pro Asp
       290                 295                 300
   Gly Leu Pro Tyr Leu Lys Val Leu Lys His Ser Gly Ile Asn Ser Ser
   305                 310                 315                 320
   Asn Ala Glu Val Leu Ala Leu Phe Asn Val Thr Glu Ala Asp Ala Gly
                   325                 330                 335
   Glu Tyr Ile Cys Lys Val Ser Asn Tyr Ile Gly Gln Ala Asn Gln Ser
               340                 345                 350
   Ala Trp Leu Thr Val Leu Pro Lys Gln Gln Ala Pro Gly Arg Glu Lys
           355                 360                 365
   Glu Ile Thr Ala Ser Pro Asp Tyr Leu Glu Ile Ala Ile Tyr Cys Ile
       370                 375                 380
   Gly Val Phe Leu Ile Ala Cys Met Val Val Thr Val Ile Leu Cys Arg
   385                 390                 395                 400
   Met Lys Asn Thr Thr Lys Lys Pro Asp Phe Ser Ser Gln Pro Ala Val
                   405                 410                 415
   His Lys Leu Thr Lys Arg Ile Pro Leu Arg Arg Gln Val Thr Val Ser
               420                 425                 430
   Ala Glu Ser Ser Ser Ser Met Asn Ser Asn Thr Pro Leu Val Arg Ile
           435                 440                 445
   Thr Thr Arg Leu Ser Ser Thr Ala Asp Thr Pro Met Leu Ala Gly Val
       450                 455                 460
   Ser Glu Tyr Glu Leu Pro Glu Asp Pro Lys Trp Glu Phe Pro Arg Asp
   465                 470                 475                 480
   Lys Leu Thr Leu Gly Lys Pro Leu Gly Glu Gly Cys Phe Gly Gln Val
                   485                 490                 495
   Val Met Ala Glu Ala Val Gly Ile Asp Lys Asp Lys Pro Lys Glu Ala
               500                 505                 510
   Val Thr Val Ala Val Lys Met Leu Lys Asp Asp Ala Thr Glu Lys Asp
           515                 520                 525
   Leu Ser Asp Leu Val Ser Glu Met Glu Met Met Lys Met Ile Gly Lys
       530                 535                 540
   His Lys Asn Ile Ile Asn Leu Leu Gly Ala Cys Thr Gln Asp Gly Pro
   545                 550                 555                 560
   Leu Tyr Val Ile Val Glu Tyr Ala Ser Lys Gly Asn Leu Arg Glu Tyr
                   565                 570                 575
   Leu Arg Ala Arg Arg Pro Pro Gly Met Glu Tyr Ser Tyr Asp Ile Asn
               580                 585                 590
   Arg Val Pro Glu Glu Gln Met Thr Phe Lys Asp Leu Val Ser Cys Thr
           595                 600                 605
   Tyr Gln Leu Ala Arg Gly Met Glu Tyr Leu Ala Ser Gln Lys Cys Ile
       610                 615                 620
   His Arg Asp Leu Ala Ala Arg Asn Val Leu Val Thr Glu Asn Asn Val
   625                 630                 635                 640
   Met Lys Ile Ala Asp Phe Gly Leu Ala Arg Asp Ile Asn Asn Ile Asp
                   645                 650                 655
   Tyr Tyr Lys Lys Thr Thr Asn Gly Arg Leu Pro Val Lys Trp Met Ala
               660                 665                 670
   Pro Glu Ala Leu Phe Asp Arg Val Tyr Thr His Gln Ser Asp Val Trp
           675                 680                 685
   Ser Phe Gly Val Leu Met Trp Glu Ile Phe Thr Leu Gly Gly Ser Pro
       690                 695                 700
   Tyr Pro Gly Ile Pro Val Glu Glu Leu Phe Lys Leu Leu Lys Glu Gly
   705                 710                 715                 720
   His Arg Met Asp Lys Pro Ala Asn Cys Thr Asn Glu Leu Tyr Met Met
                   725                 730                 735
   Met Arg Asp Cys Trp His Ala Val Pro Ser Gln Arg Pro Thr Phe Lys
               740                 745                 750
   Gln Leu Val Glu Asp Leu Asp Arg Ile Leu Thr Leu Thr Thr Asn Glu
           755                 760                 765
   Glu Tyr Leu Asp Leu Ser Gln Pro Leu Glu Gln Tyr Ser Pro Ser Tyr
       770                 775                 780
   Pro Asp Thr Arg Ser Ser Cys Ser Ser Gly Asp Asp Ser Val Phe Ser
   785                 790                 795                 800
   Pro Asp Pro Met Pro Tyr Glu Pro Cys Leu Pro Gln Tyr Pro His Ile
                   805                 810                 815
   Asn Gly Ser Val Lys Thr
               820

## Description

Antibody based cancer therapies have been successfully introduced into the clinic and have emerged as the most promising therapeutics in oncology over the last decade. Antibody-based therapies for cancer have the potential of higher specificity and lower side effect profile as compared to conventional drugs.

Targets for antibody-based therapies are, in particular, molecular structures which are exclusively expressed or overexpressed in tumor cells. Moreover, if a molecular structure is functionally involved in cancer development, binding of antibodies to this structure can antagonize its function resulting in a therapeutically beneficial effect.

PLAC1 is a placenta-specific gene which is frequently aberrantly activated and highly expressed in a variety of tumor types. PLAC1 is localized on the surface of cancer cells and is accessible for antibodies. In the case of breast cancer PLAC1 is expressed in 82 % of the patients. In the case of lung cancer and gastric cancer, PLAC1 is expressed in 42 and 58%, respectively, of the cases.

Massabbal E. et al. (2005) Mol. Reprod. Dev. 71:299-304 describe that PLAC1 expression is upregulated during trophoblast differentiation, localizing primarily to the differentiated syncytiotrophoblast and that PLAC1 expression is specifically regulated by peptide growth factors relevant to trophoblast differentiation.

WO 2012/007981 describes the use of anti-PLAC1 protein antibodies as biomarkers of infertility, diagnostic kit for the detection of the immune response against PLAC1 and use of PLAC1 protein in therapeutic and contraceptive fields.

It has been observed that PLAC1 interacts with FGF7 and that PLAC1, FGF7 and FGFR2IIIb can form a trimeric complex. Furthermore, it has been observed that PLAC1 acts as co-factor for FGF7-mediated specific phosphorylation of FGFR2IIIb thus enhancing proliferation and migration of cells.

### SUMMARY OF THE INVENTION

The present invention generally provides antibodies and antibody mixtures useful as therapeutics for treating and/or preventing cancer diseases, in particular cancer diseases associated with cells expressing PLAC1 and/or being characterized by association of PLAC1 with their cell surface. Such cancer diseases include breast cancer, lung cancer, gastric cancer, ovarian cancer, hepatocellular cancer, colon cancer, pancreatic cancer, esophageal cancer, head & neck cancer, kidney cancer, in particular renal cell carcinoma, prostate cancer, liver cancer, melanoma, sarcoma, myeloma, neuroblastoma, placental choriocarcinoma, cervical cancer, and thyroid cancer, and the metastatic forms thereof.

In one aspect the invention provides a monoclonal antibody or a mixture of monoclonal antibodies which binds to the C-terminus of PLAC1, preferably to the amino acid sequence spanning from amino acids 23 to 212 of PLAC1 and blocks the interaction between PLAC1 and FGF7 for use in a method for treating cancer or for use in a method for treating cancer metastasis, wherein PLAC1 has the the amino acid sequence of SEQ ID NO: 1. The antibody or mixture of antibodies binds to PLAC1. In one embodiment, the antibody or mixture of antibodies is capable of immunoprecipitating PLAC1. The antibody of the invention is a monoclonal antibody. Also described herein is a mixture of antibodies which is a polyclonal antibody. In one embodiment, the mixture of antibodies of the invention is a mixture of two or more monoclonal antibodies. In one embodiment, the mixture of antibodies of the invention comprises two or more antibodies each of said two or more antibodies binding to different epitopes of PLAC1. In one embodiment, the mixture of antibodies of the invention comprises one or more antibodies binding to PLAC1 and (i) one or more antibodies binding to FGF7 and/or (ii) one or more antibodies binding to FGFR2IIIb. In one embodiment, the antibody or mixture of antibodies of the invention inhibits FGF7-dependent phosphorylation of FGFR2IIIb and/or FGF7/FGFR2IIIb-dependent phosphorylation of Akt. Preferably, said phosphorylation of Akt takes place at Thr308 and/or Ser473. In one embodiment, the antibody or mixture of antibodies of the invention inhibits proliferation, migration and/or attachment of cancer cells, preferably cancer cells expressing PLAC1. In one embodiment, said proliferation, migration and/or attachment of cancer cells is FGF7/FGFR2IIIb-dependent.

The antibodies and antibody mixtures described herein preferably block the interaction between PLAC1 and FGF7 by binding to an epitope present on PLAC1, more preferably by binding to an epitope present on PLAC1 which is involved in the interaction with FGF7.

Preferably, blocking the interaction between PLAC1 and FGF7 results in inhibition of proliferation, migration and/or attachment of cells, in particular cells expressing PLAC1 and/or being characterized by association of PLAC1 with their cell surface, such as cancer cells. Such inhibition of proliferation, migration and/or attachment of cells is preferably induced by binding of the antibody or mixture of antibodies to PLAC1 expressed by said cells and/or being associated with the cell surface of said cells and can be utilized therapeutically as described herein. In particular, inhibition of proliferation of cells can be utilized for treating or preventing cancer. Inhibition of proliferation, migration and/or attachment of cells can be utilized, in particular, for treating or preventing cancer metastasis and the metastatic spread of cancer cells.

In one embodiment, the antibodies and mixtures of antibodies described herein inhibit proliferation, migration and/or attachment of cells expressing PLAC1 and/or being characterized by association of PLAC1 with their cell surface. Preferably, the antibodies and mixtures of antibodies described herein do not inhibit proliferation, migration and/or attachment of cells not expressing PLAC1 and/or not being characterized by association of PLAC1 with their cell surface.

According to the invention, the cells mentioned herein such as cells expressing PLAC1 and/or being characterized by association of PLAC1 with their cell surface are preferably cancer cells and are, in particular, selected from the group consisting of tumorigenic cancer cells of the following cancer diseases: breast cancer, lung cancer, gastric cancer, ovarian cancer, hepatocellular cancer, colon cancer, pancreatic cancer, esophageal cancer, head & neck cancer, kidney cancer, in particular renal cell carcinoma, prostate cancer, liver cancer, melanoma, sarcoma, myeloma, neuroblastoma, placental choriocarcinoma, cervical cancer, and thyroid cancer, and the metastatic forms thereof.

In one particularly preferred embodiment, the antibodies or mixtures of antibodies described herein have the activity of inhibiting proliferation of cells such as cells expressing PLAC1 and/or being characterized by association of PLAC1 with their cell surface, preferably cancer cells. This activity can be measured in vitro by determining proliferation of cells in an assay using bromodeoxyuridine (5-bromo-2-deoxyuridine, BrdU). BrdU is a synthetic nucleoside which is an analogue of thymidine and can be incorporated into the newly synthesized DNA of replicating cells (during the S phase of the cell cycle), substituting for thymidine during DNA replication. Detecting the incorporated chemical using, for example, antibodies specific for BrdU indicates cells that were actively replicating their DNA. The cell lines BT-549, Caov-3, EFO-21, SK-BR-3, MCF-7, MDA-MB-468 and MDA-MB-231 can be used as PLAC1-expressing cancer cells.

Blocking the interaction between PLAC1 and FGF7 by binding of an antibody or a mixture of antibodies of the invention to the PLAC1 antigen may inhibit proliferation of cells such as cells expressing PLAC1 and/or being characterized by association of PLAC1 with their cell surface (e.g. cancer cells). Alternatively or in addition to inhibiting proliferation of cells, binding of an antibody or a mixture of antibodies of the invention to the PLAC1 antigen may inhibit migration and/or attachment of cells such as cells expressing PLAC1 and/or being characterized by association of PLAC1 with their cell surface (e.g. cancer cells), and thus, may inhibit metastatic spread of cancer cells.

According to all aspects of the invention, PLAC1 is preferably human PLAC1, preferably having the amino acid sequence according to SEQ ID NO: 1, more preferably having the amino acid sequence spanning from amino acids 23 to 212 of SEQ ID NO: 1.

The antibodies or mixtures of antibodies described herein may be obtained by a method comprising the step of immunizing an animal with a protein having the amino acid sequence according to SEQ ID NO: 1, or an immunogenic fragment thereof, or a nucleic acid or host cell expressing said protein or immunogenic fragment.

Preferably, the antibodies or mixtures of antibodies described herein are specific for PLAC1.

In one embodiment, antibodies described herein such as an antibodies of the invention or antibodies comprised in a mixture of antibodies of the invention are functionally linked (e.g., by chemical coupling, genetic fusion, noncovalent association or otherwise) to one or more other molecular entities, such as another antibody (e.g. to produce a bispecific or a multispecific antibody), a cellular ligand or a therapeutic moiety such as a toxin, a radioisotope, a drug such as a small molecule anti-cancer drug, a recombinant cytokine or chemokine, or a cytotoxic agent. Accordingly, the present invention encompasses a large variety of antibody conjugates, bispecific and multispecific molecules, and fusion proteins. Generally, for the purposes of the present invention, all antibody derivatives such as antibody conjugates, bispecific and multispecific molecules, and fusion proteins described herein are encompassed by the term "antibody".

Antibodies described herein may be derived from different species, including but not limited to mouse, rat, rabbit, guinea pig and human.

Antibodies described herein also include chimeric molecules in which an antibody constant region derived from one species, preferably human, is combined with the antigen binding site derived from another species. Moreover, antibodies described herein include humanized molecules in which the antigen binding sites of an antibody derived from a non-human species are combined with constant and framework regions of human origin.

Antibodies described herein include polyclonal and monoclonal antibodies and include IgA such as IgA1 or IgA2, IgG1, IgG2, IgG3, IgG4, IgE, IgM, and IgD antibodies. In various embodiments, the antibody is an IgG1 antibody, more particularly an IgG1, kappa or IgG1, lambda isotype (i.e. IgG1, κ, λ), an IgG2a antibody (e.g. IgG2a, κ, λ), an IgG2b antibody (e.g. IgG2b, κ, λ), an IgG3 antibody (e.g. IgG3, κ, λ) or an IgG4 antibody (e.g. IgG4, κ, λ). The antibodies can be whole antibodies or antigen-binding fragments thereof including, for example, Fab, F(ab')₂, Fv, single chain Fv fragments or bispecific antibodies. Furthermore, the antigen-binding fragments include binding-domain immunoglobulin fusion proteins comprising (i) a binding domain polypeptide (such as a heavy chain variable region or a light chain variable region) that is fused to an immunoglobulin hinge region polypeptide, (ii) an immunoglobulin heavy chain CH2 constant region fused to the hinge region, and (iii) an immunoglobulin heavy chain CH3 constant region fused to the CH2 constant region. Such binding-domain immunoglobulin fusion proteins are further disclosed in US2003/0118592 and US 2003/0133939. The antibodies described herein may be chimeric, human, or humanized antibodies.

In preferred embodiments, the antibodies described herein can be characterized by one or more of the following properties:
a) specificity for PLAC1;
b) a binding affinity to PLAC1 of about 100 nM or less, preferably, about 5-10 nM or less and, more preferably, about 1-3 nM or less;
c) the ability to inhibit the growth of cells such as cells which express PLAC1 and/or cells which are characterized by association of PLAC1 with their cell surface;
d) the ability to inhibit migration of cells such as cells which express PLAC1 and/or cells which are characterized by association of PLAC1 with their cell surface;
e) the ability to inhibit attachment of cells such as cells which express PLAC1 and/or cells which are characterized by association of PLAC1 with their cell surface.

In another aspect, the invention provides compositions, e.g., pharmaceutical compositions/kits, comprising a monoclonal antibody or a mixture of monoclonal antibodies which binds to the C-terminus of PLAC1, preferably to the amino acid sequence spanning from amino acids 23 to 212 of PLAC1 and blocks the interaction between PLAC1 and FGF7 for use in a method for treating cancer or for use in treating cancer metatstasis, wherein PLAC1 has the amino acid sequence of SEQ ID NO: 1. A pharmaceutical composition of the invention may comprise a pharmaceutically acceptable carrier and may optionally comprise one or more adjuvants, stabilizers etc.

A pharmaceutical composition of the invention is for use in a method for treating cancer or for use in a method for treating cancer metastasis. In one embodiment, the cancer cells express PLAC1.

Antibodies, mixtures of antibodies and compositions described herein may be used to treat and/or prevent a variety of diseases preferably diseases involving cells expressing PLAC1 and/or being characterized by association of PLAC1 with their cell surface by administering the antibodies, mixtures of antibodies and compositions to patients suffering from such diseases.

In this aspect, the present disclosure describes methods for the treatment or prevention of diseases, in particular cancer diseases, by blocking the interaction between PLAC1 and FGF7, in particular through targeting PLAC1 such as PLAC1 expressed by cells and/or being associated with the surface of cells, such as cancer cells. Preferred diseases for a treatment or prevention are those in which cells expressing PLAC1 and/or being characterized by association of PLAC1 with their cell surface are involved such as cancer diseases, in particular cancer diseases as described herein.

Specifically, the antibodies, mixtures of antibodies and compositions described herein may be used in a variety of methods for inhibiting growth of cells such as cells expressing PLAC1 and/or being characterized by association of PLAC1 with their cell surface and/or inhibiting metastatic spread of cells such as cells expressing PLAC1 and/or being characterized by association of PLAC1 with their cell surface by contacting the cells with an effective amount of the antibodies, mixtures of antibodies and compositions, such that the growth of the cells and/or the metastatic spread of the cells is inhibited. Cells expressing PLAC1 and/or being characterized by association of PLAC1 with their cell surface include cancer cells.

Exemplary diseases that can be treated (e.g., ameliorated) or prevented according to the present disclosure include, but are not limited to, cancer diseases. Examples of cancer diseases, which can be treated and/or prevented, include breast cancer, lung cancer, gastric cancer, ovarian cancer, hepatocellular cancer, colon cancer, pancreatic cancer, esophageal cancer, head & neck cancer, kidney cancer, in particular renal cell carcinoma, prostate cancer, liver cancer, melanoma, sarcoma, myeloma, neuroblastoma, placental choriocarcinoma, cervical cancer, and thyroid cancer, and the metastatic forms thereof.

Accordingly, the present disclosure describes a method for treating cancer comprising the step of administering the antibody or mixture of antibodies of the invention. Furthermore, the present disclosure describes a method for treating cancer metastasis comprising the step of administering the antibody or mixture of antibodies of the invention. In one embodiment, the cancer cells express PLAC1.

The present invention provides the antibodies, mixtures of antibodies and compositions described herein for use in a method for treating cancer or for use in a method for treating cancer metastasis.

Other features and advantages of the instant invention will be apparent from the following detailed description and claims.

### BRIEF DESCRIPTION OF THE DRAWINGS

Figure 1: Distinct mixtures of PLAC1 antibodies recognizing different epitopes can much better bind and precipitate native PLAC1 than other mixtures.
Figure 2: PLAC1 specific monoclonal antibodies block the binding of PLAC1 to FGF7 in an in vitro interaction assay using recombinant FGF7 and overexpressed PLAC1.
Figure 3: Co-immunoprecipitation assay demonstrating that PLAC1 interacts specifically with FGF7 and forms a complex with the FGFR2IIIb receptor in vivo.
Figure 4: Using microarray analyses and Western blotting the mechanism of action of PLAC1 was analyzed and characterized. Cell proliferation is controlled by FGFR2IIIb dependent phosphorylation and activation of Akt in BeWo choriocarcinoma cells.
Figure 5: FGF7 dependent stimulation of Akt is influenced by PLAC1 in MCF7 cells.
Figure 6: PLAC1 is required for the FGF7/FGFR2IIIb-mediated proliferation in the breast cancer cell line MCF7.
Figure 7: PLAC1 is required for the FGF7-mediated proliferation in the choriocarcinoma cell line BeWo.
Figure 8: hPLAC1 promotes attachment of the choriocarcinoma cell line BeWo to cell culture surfaces.
Figure 9: PLAC1 is required for the attachment of HEK cells to surfaces.

### DEFINITION OF TERMS

Unless defined otherwise, all technical and scientific terms used herein have the same meanings as commonly understood by one of ordinary skill in the art.

In the following, the elements of the present invention will be described.

Preferably, the terms used herein are defined as described in "A multilingual glossary of biotechnological terms: (IUPAC Recommendations)", H.G.W. Leuenberger, B. Nagel, and H. Kölbl, Eds., Helvetica Chimica Acta, CH-4010 Basel, Switzerland, (1995).

The practice of the present invention will employ, unless otherwise indicated, conventional methods of chemistry, biochemistry, cell biology, immunology, and recombinant DNA techniques which are explained in the literature in the field (cf., e.g., Molecular Cloning: A Laboratory Manual, 2nd Edition, J. Sambrook et al. eds., Cold Spring Harbor Laboratory Press, Cold Spring Harbor 1989).

Throughout this specification and the claims which follow, unless the context requires otherwise, the word "comprise", and variations such as "comprises" and "comprising", will be understood to imply the inclusion of a stated member, integer or step or group of members, integers or steps but not the exclusion of any other member, integer or step or group of members, integers or steps although in some embodiments such other member, integer or step or group of members, integers or steps may be excluded, i.e. the subject-matter consists in the inclusion of a stated member, integer or step or group of members, integers or steps. The terms "a" and "an" and "the" and similar reference used in the context of describing the invention (especially in the context of the claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as"), provided herein is intended merely to better illustrate the invention and does not pose a limitation on the scope of the invention otherwise claimed. No language in the specification should be construed as indicating any non-claimed element essential to the practice of the invention.

The term "PLAC1" preferably relates to human PLAC1, and, in particular, to a protein comprising the amino acid sequence according to SEQ ID NO: 1 of the sequence listing or a variant of said amino acid sequence.

In one embodiment, the term "PLAC1" relates to the amino acid sequence of PLAC1 not including the N-terminal hydrophobic domain. The term "PLAC1" includes a protein comprising amino acids 29 to 119, preferably amino acids 29 to 212 and more preferably amino acids 23 to 212 of SEQ ID NO: 1 or the corresponding amino acids of a variant of SEQ ID NO: 1.

FGF7 is a member of the fibroblast growth factor (FGF) family. FGF family members possess broad mitogenic and cell survival activities, and are involved in a variety of biological processes, including embryonic development, cell growth, morphogenesis, tissue repair, tumor growth and invasion.

The term "FGF7" preferably relates to human FGF7, and, in particular, to a protein comprising the amino acid sequence according to SEQ ID NO: 2 of the sequence listing or a variant of said amino acid sequence.

FGFR2 is a receptor for fibroblast growth factor. FGFR family members differ from one another in their ligand affinities and tissue distribution. A full-length representative protein consists of an extracellular region, composed of three immunoglobulin domains, a single hydrophobic membrane-spanning segment and a cytoplasmic tyrosine kinase domain. The extracellular portion of the protein interacts with fibroblast growth factors, setting in motion a cascade of downstream signals, ultimately influencing mitogenesis and differentiation. FGFR2 has two naturally occurring isoforms FGFR2IIIb and FGFR2IIIc, created by splicing of the third immunoglobulin-like domain. FGFR2IIIb binds to FGF-1, -3, -7, -10, -22.

The term "FGFR2IIIb" preferably relates to human FGFR2IIIb, and, in particular, to a protein comprising the amino acid sequence according to SEQ ID NO: 3 of the sequence listing or a variant of said amino acid sequence.

The term "variant" according to the invention refers, in particular, to mutants, splice variants, conformations, isoforms, allelic variants, species variants and species homologs, in particular those which are naturally present. An allelic variant relates to an alteration in the normal sequence of a gene, the significance of which is often unclear. Complete gene sequencing often identifies numerous allelic variants for a given gene. A species homolog is a nucleic acid or amino acid sequence with a different species of origin from that of a given nucleic acid or amino acid sequence.

The term "variant" shall encompass any posttranslationally modified variants and conformation variants.

"Cell surface" is used in accordance with its normal meaning in the art, and thus includes the outside of the cell which is accessible to binding by proteins and other molecules.

PLAC1 is associated with the surface of cells if it is located at the surface of said cells and is accessible to binding by PLAC1-specific antibodies added to the cells. In preferred embodiments, a cell being characterized by association of PLAC1 with its cell surface is a cell expressing PLAC1. It is to be understood that in the case where PLAC1 is expressed by cells, the PLAC1 associated with the surface of said cells may only be a portion of the expressed PLAC1, in particular a portion thereof as defined above.

According to the invention PLAC1 is not substantially expressed in a cell and/or is not substantially associated with a cell surface if the level of expression and/or association is lower compared to expression and/or association in placenta cells or placenta tissue. Preferably, the level of expression and/or association is less than 10%, preferably less than 5%, 3%, 2%, 1%, 0.5%, 0.1% or 0.05% of the expression and/or association in placenta cells or placenta tissue or even lower. Preferably, PLAC1 is not substantially expressed in a cell and/or is not substantially associated with a cell surface if the level of expression and/or association exceeds the level of expression and/or association in non-cancerous tissue other than placenta such as non-cancerous tissue of one or more of brain, lung, breast, colon, liver, stomach, kidney, prostate, pancreas, ovary, spleen, skin, myocard, and endometrium by no more than 2-fold, preferably 1,5-fold, and preferably does not exceed the level of expression and/or association in said non-cancerous tissue(s). Preferably, PLAC1 is not substantially expressed in a cell and/or is not substantially associated with a cell surface if the level of expression or association is below the detection limit and/or if the level of expression or association is too low to allow binding by PLAC1-specific antibodies added to the cells.

According to the invention PLAC1 is expressed in a cell and/or is associated with a cell surface if the level of expression and/or association exceeds the level of expression and/or association in non-cancerous tissue other than placenta such as non-cancerous tissue of one or more of brain, lung, breast, colon, liver, stomach, kidney, prostate, pancreas, ovary, spleen, skin, myocard, and endometrium, preferably by more than 2-fold, preferably 10-fold, 100-fold, 1000-fold, or 10000-fold. Preferably, PLAC1 is expressed in a cell and/or is associated with a cell surface if the level of expression or association is above the detection limit and/or if the level of expression or association is high enough to allow binding by PLAC1-specific antibodies added to the cells. Preferably, PLAC1 expressed in a cell is expressed or exposed on the surface of said cell.

According to the invention, the term "disease" refers to any pathological state, including cancer, in particular those forms of cancer described herein.

"Diseases associated with cells expressing PLAC1 and/or being characterized by association of PLAC1 with their cell surface" means according to the invention that expression and/or association in cells of a diseased tissue or organ is preferably increased compared to the state in a healthy tissue or organ. An increase refers to an increase by at least 10%, in particular at least 20%, at least 50%, at least 100%, at least 200%, at least 500%, at least 1000%, at least 10000% or even more. In one embodiment, expression and/or association with the cell surface is only found in a diseased tissue, while expression and/or association with the cell surface in a healthy tissue is repressed. According to the invention, diseases associated with cells expressing PLAC1 and/or being characterized by association of PLAC1 with their cell surface include cancer diseases. Furthermore, according to the invention, cancer diseases preferably are those wherein the cancer cells express PLAC1 and/or are characterized by association of PLAC1 with their cell surface.

As used herein, a "cancer disease" or "cancer" includes a disease characterized by aberrantly regulated cellular growth, proliferation, differentiation, adhesion, and/or migration. By "cancer cell" is meant an abnormal cell that grows by a rapid, uncontrolled cellular proliferation and continues to grow after the stimuli that initiated the new growth cease. Preferably, a "cancer disease" is characterized by cells expressing PLAC1 and/or being characterized by association of PLAC1 with their cell surface and a cancer cell expresses PLAC1 and/or is characterized by association of PLAC1 with its cell surface. A cell expressing PLAC1 and/or being characterized by association of PLAC1 with its cell surface preferably is a cancer cell, preferably of the cancers described herein. Preferably, such cell is a cell other than a placental cell.

Preferred cancer diseases or cancers according to the invention are selected from the group consisting of breast cancer, lung cancer, gastric cancer, ovarian cancer, hepatocellular cancer, colon cancer, pancreatic cancer, esophageal cancer, head & neck cancer, kidney cancer, in particular renal cell carcinoma, prostate cancer, liver cancer, melanoma, sarcoma, myeloma, neuroblastoma, placental choriocarcinoma, cervical cancer, and thyroid cancer, and the metastatic forms thereof.

According to the invention, a "carcinoma" is a cancer that begins in the lining layer (epithelial cells) of organs.

Choriocarcinoma is a malignant, trophoblastic and aggressive cancer, usually of the placenta. It is characterized by early hematogenous spread to the lungs.

A sarcoma is a cancer of the connective tissue (bone, cartilage, fat) resulting in mesoderm proliferation. This is in contrast to carcinomas, which are of epithelial origin.

Renal cell carcinoma also known as renal cell cancer or renal cell adenocarcinoma is a kidney cancer that originates in the lining of the proximal convoluted tubule, the very small tubes in the kidney that filter the blood and remove waste products. Renal cell carcinoma is by far the most common type of kidney cancer in adults and the most lethal of all the genitorurinary tumors. Distinct subtypes of renal cell carcinoma are clear cell renal cell carcinoma and papillary renal cell carcinoma. Clear cell renal cell carcinoma is the most common form of renal cell carcinoma. When seen under a microscope, the cells that make up clear cell renal cell carcinoma appear very pale or clear. Papillary renal cell carcinoma is the second most common subtype. These cancers form little finger-like projections (called papillae) in some, if not most, of the tumors.

By "metastasis" is meant the spread of cancer cells from its original site to another part of the body. The formation of metastasis is a very complex process and depends on detachment of malignant cells from the primary tumor, invasion of the extracellular matrix, penetration of the endothelial basement membranes to enter the body cavity and vessels, and then, after being transported by the blood, infiltration of target organs. Finally, the growth of a new tumor at the target site depends on angiogenesis. Tumor metastasis often occurs even after the removal of the primary tumor because tumor cells or components may remain and develop metastatic potential. In one embodiment, the term "metastasis" according to the invention relates to "distant metastasis" which relates to a metastasis which is remote from the primary tumor and the regional lymph node system.

The cells of a secondary or metastatic tumor are like those in the original tumor. This means, for example, that, if ovarian cancer metastasizes to the liver, the secondary tumor is made up of abnormal ovarian cells, not of abnormal liver cells. The tumor in the liver is then called metastatic ovarian cancer, not liver cancer.

In one embodiment, a cancer according to the invention is metastatic breast cancer, preferably metastatic breast cancer in the lung.

By "treat" is meant to administer a compound or composition to a subject in order to prevent or eliminate a disease, including reducing the size of a tumor or the number of tumors in a subject; arrest or slow a disease in a subject; inhibit or slow the development of a new disease in a subject; decrease the frequency or severity of symptoms and/or recurrences in a subject who currently has or who previously has had a disease; and/or prolong, i.e. increase the lifespan of the subject.

In particular, the term "treatment of a disease" includes curing, shortening the duration, ameliorating, preventing, slowing down or inhibiting progression or worsening, or preventing or delaying the onset of a disease or the symptoms thereof.

The term "patient" means according to the invention a human being, a nonhuman primate or another animal, in particular a mammal such as a cow, horse, pig, sheep, goat, dog, cat or a rodent such as a mouse and rat. In a particularly preferred embodiment, the patient is a human being.

The term "antigen" relates to an agent such as a protein or peptide comprising an epitope against which an immune response is directed and/or is to be directed. In a preferred embodiment, an antigen is a tumor-associated antigen, such as PLAC1, i.e., a constituent of cancer cells which may be derived from the cytoplasm, the cell surface and the cell nucleus, in particular those antigens which are produced, preferably in large quantity, intracellular or as surface antigens on cancer cells.

In the context of the present invention, the term "tumor-associated antigen" preferably relates to proteins that are under normal conditions specifically expressed in a limited number of tissues and/or organs or in specific developmental stages, for example, the tumor-associated antigen may be under normal conditions specifically expressed in reproductive organs, e.g., in testis, in trophoblastic tissue, e.g., in placenta, or in germ line cells, and are expressed or aberrantly expressed in one or more tumor or cancer tissues. The tumor-associated antigens in the context of the present invention include, for example, differentiation antigens, preferably cell type specific differentiation antigens, i.e., proteins that are under normal conditions specifically expressed in a certain cell type at a certain differentiation stage, cancer/testis antigens, i.e., proteins that are under normal conditions specifically expressed in testis and sometimes in placenta, and germ line specific antigens. In the context of the present invention, the tumor-associated antigen is preferably associated with the cell surface of a cancer cell and is preferably not or only rarely expressed in normal tissues.

The term "epitope" refers to an antigenic determinant in a molecule, i.e., to the part in a molecule that is recognized by the immune system, for example, that is recognized by an antibody. For example, epitopes are the discrete, three-dimensional sites on an antigen, which are recognized by the immune system. Epitopes usually consist of chemically active surface groupings of molecules such as amino acids or sugar side chains and usually have specific three dimensional structural characteristics, as well as specific charge characteristics. Conformational and non-conformational epitopes are distinguished in that the binding to the former but not the latter is lost in the presence of denaturing solvents. An epitope of a protein such as PLAC1 preferably comprises a continuous or discontinuous portion of said protein and is preferably between 5 and 100, preferably between 5 and 50, more preferably between 8 and 30, most preferably between 10 and 25 amino acids in length, for example, the epitope may be preferably 8, 9, 10, 11, 12, 13, 14, 15, 16, 17, 18, 19, 20, 21, 22, 23, 24, or 25 amino acids in length.

The term "antibody" refers to a glycoprotein comprising at least two heavy (H) chains and two light (L) chains inter-connected by disulfide bonds, and includes any molecule comprising an antigen binding portion thereof. The term "antibody" includes monoclonal antibodies and fragments or derivatives of antibodies, including, without limitation, human antibodies, humanized antibodies, chimeric antibodies, single chain antibodies, e.g., scFv's and antigen-binding antibody fragments such as Fab and Fab' fragments and also includes all recombinant forms of antibodies, e.g., antibodies expressed in prokaryotes, unglycosylated antibodies, and any antigen-binding antibody fragments and derivatives as described herein. Each heavy chain is comprised of a heavy chain variable region (abbreviated herein as VH) and a heavy chain constant region. Each light chain is comprised of a light chain variable region (abbreviated herein as VL) and a light chain constant region. The VH and VL regions can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (C1q) of the classical complement system.

The antibodies described herein may be human antibodies. The term "human antibody", as used herein, is intended to include antibodies having variable and constant regions derived from human germline immunoglobulin sequences. The human antibodies described herein may include amino acid residues not encoded by human germline immunoglobulin sequences (e.g., mutations introduced by random or site-specific mutagenesis *in vitro* or by somatic mutation *in vivo*).

The term "humanized antibody" refers to a molecule having an antigen binding site that is substantially derived from an immunoglobulin from a non-human species, wherein the remaining immunoglobulin structure of the molecule is based upon the structure and/or sequence of a human immunoglobulin. The antigen binding site may either comprise complete variable domains fused onto constant domains or only the complementarity determining regions (CDR) grafted onto appropriate framework regions in the variable domains. Antigen binding sites may be wild-type or modified by one or more amino acid substitutions, e.g. modified to resemble human immunoglobulins more closely. Some forms of humanized antibodies preserve all CDR sequences (for example a humanized mouse antibody which contains all six CDRs from the mouse antibody). Other forms have one or more CDRs which are altered with respect to the original antibody.

The term "chimeric antibody" refers to those antibodies wherein one portion of each of the amino acid sequences of heavy and light chains is homologous to corresponding sequences in antibodies derived from a particular species or belonging to a particular class, while the remaining segment of the chain is homologous to corresponding sequences in another. Typically the variable region of both light and heavy chains mimics the variable regions of antibodies derived from one species of mammals, while the constant portions are homologous to sequences of antibodies derived from another. One clear advantage to such chimeric forms is that the variable region can conveniently be derived from presently known sources using readily available B-cells or hybridomas from non-human host organisms in combination with constant regions derived from, for example, human cell preparations. While the variable region has the advantage of ease of preparation and the specificity is not affected by the source, the constant region being human, is less likely to elicit an immune response from a human subject when the antibodies are injected than would the constant region from a non human source. However the definition is not limited to this particular example.

The terms "antigen-binding portion" of an antibody (or simply "binding portion") or "antigen-binding fragment" of an antibody (or simply "binding fragment") or similar terms refer to one or more fragments of an antibody that retain the ability to specifically bind to an antigen. It has been shown that the antigen-binding function of an antibody can be performed by fragments of a full-length antibody. Examples of binding fragments encompassed within the term "antigen-binding portion" of an antibody include (i) Fab fragments, monovalent fragments consisting of the VL, VH, CL and CH domains; (ii) F(ab')₂ fragments, bivalent fragments comprising two Fab fragments linked by a disulfide bridge at the hinge region; (iii) Fd fragments consisting of the VH and CH domains; (iv) Fv fragments consisting of the VL and VH domains of a single arm of an antibody, (v) dAb fragments (Ward et al., (1989) Nature 341: 544-546), which consist of a VH domain; (vi) isolated complementarity determining regions (CDR), and (vii) combinations of two or more isolated CDRs which may optionally be joined by a synthetic linker. Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see e.g., Bird et al. (1988) Science 242: 423-426; and Huston et al. (1988) Proc. Natl. Acad. Sci. USA 85: 5879-5883). Such single chain antibodies are also intended to be encompassed within the term "antigen-binding fragment" of an antibody. A further example is binding-domain immunoglobulin fusion proteins comprising (i) a binding domain polypeptide that is fused to an immunoglobulin hinge region polypeptide, (ii) an immunoglobulin heavy chain CH2 constant region fused to the hinge region, and (iii) an immunoglobulin heavy chain CH3 constant region fused to the CH2 constant region. The binding domain polypeptide can be a heavy chain variable region or a light chain variable region. The binding-domain immunoglobulin fusion proteins are further disclosed in US 2003/0118592 and US 2003/0133939. These antibody fragments are obtained using conventional techniques known to those with skill in the art, and the fragments are screened for utility in the same manner as are intact antibodies.

The term "bispecific molecule" is intended to include any agent, e.g., a protein, peptide, or protein or peptide complex, which has two different binding specificities. For example, the molecule may bind to, or interact with (a) a cell surface antigen, and (b) an Fc receptor on the surface of an effector cell. The term "multispecific molecule" or "heterospecific molecule" is intended to include any agent, e.g., a protein, peptide, or protein or peptide complex, which has more than two different binding specificities. For example, the molecule may bind to, or interact with (a) a cell surface antigen, (b) an Fc receptor on the surface of an effector cell, and (c) at least one other component. Accordingly, the invention includes, but is not limited to, bispecific, trispecific, tetraspecific, and other multispecific molecules which are directed to PLAC1, and to other targets, such as Fc receptors on effector cells. The term "bispecific antibodies" also includes diabodies. Diabodies are bivalent, bispecific antibodies in which the VH and VL domains are expressed on a single polypeptide chain, but using a linker that is too short to allow for pairing between the two domains on the same chain, thereby forcing the domains to pair with complementary domains of another chain and creating two antigen binding sites (see e.g. , Holliger, P., et al. (1993) Proc. Natl. Acad. Sci. USA 90: 6444-6448; Poljak, R. J., et al. (1994) Structure 2: 1121-1123).

An antibody may be conjugated to a therapeutic moiety or agent, such as a cytotoxin, a drug (e.g., an immunosuppressant) or a radioisotope. A cytotoxin or cytotoxic agent includes any agent that is detrimental to and, in particular, kills cells. Examples include taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof. Suitable therapeutic agents for forming antibody conjugates include, but are not limited to, antimetabolites (e.g., methotrexate, 6-mercaptopurine, 6-thioguanine, cytarabine, fludarabin, 5-fluorouracil decarbazine), alkylating agents (e.g., mechlorethamine, thioepa chlorambucil, melphalan, carmustine (BSNU) and lomustine (CCNU), cyclophosphamide, busulfan, dibromomannitol, streptozotocin, mitomycin C, and cis-dichlorodiamine platinum (II) (DDP) cisplatin), anthracyclines (e.g., daunorubicin (formerly daunomycin) and doxorubicin), antibiotics (e.g., dactinomycin (formerly actinomycin), bleomycin, mithramycin, and anthramycin (AMC), and anti-mitotic agents (e.g., vincristine and vinblastine). In a preferred embodiment, the therapeutic agent is a cytotoxic agent or a radiotoxic agent. In another embodiment, the therapeutic agent is an immunosuppressant. In yet another embodiment, the therapeutic agent is GM-CSF. In a preferred embodiment, the therapeutic agent is doxorubicin, cisplatin, bleomycin, sulfate, carmustine, chlorambucil, cyclophosphamide or ricin A.

Antibodies also can be conjugated to a radioisotope, e.g., iodine-131, yttrium-90 or indium-111, to generate cytotoxic radiopharmaceuticals.

The antibody conjugates of the invention can be used to modify a given biological response, and the drug moiety is not to be construed as limited to classical chemical therapeutic agents. For example, the drug moiety may be a protein or polypeptide possessing a desired biological activity. Such proteins may include, for example, an enzymatically active toxin, or active fragment thereof, such as abrin, ricin A, pseudomonas exotoxin, or diphtheria toxin; a protein such as tumor necrosis factor or interferon-y; or, biological response modifiers such as, for example, lymphokines, interleukin-1 ("IL-1"), interleukin-2 ("IL-2"), interleukin-6 ("IL-6"), granulocyte macrophage colony stimulating factor ("GM-CSF"), granulocyte colony stimulating factor ("G-CSF"), or other growth factors.

Techniques for conjugating such therapeutic moiety to antibodies are well known, see, e.g., Arnon et al., "Monoclonal Antibodies For Immunotargeting Of Drugs In Cancer Therapy", in Monoclonal Antibodies And Cancer Therapy, Reisfeld et al. (eds.), pp. 243-56 (Alan R. Liss, Inc. 1985); Hellstrom et al., "Antibodies For Drug Delivery", in Controlled Drug Delivery (2nd Ed.), Robinson et al. (eds.), pp. 623-53 (Marcel Dekker, Inc. 1987); Thorpe, "Antibody Carriers Of Cytotoxic Agents In Cancer Therapy: A Review", in Monoclonal Antibodies '84: Biological And Clinical Applications, Pincheraet al. (eds.), pp. 475-506 (1985); "Analysis, Results, And Future Prospective Of The Therapeutic Use Of Radiolabeled Antibody In Cancer Therapy", in Monoclonal Antibodies For Cancer Detection And Therapy, Baldwin et al. (eds.), pp. 303-16 (Academic Press 1985), and Thorpe et al., "The Preparation And Cytotoxic Properties Of Antibody-Toxin Conjugates", Immunol. Rev., 62: 119-58 (1982).

As used herein, an antibody is "derived from" a particular germline sequence if the antibody is obtained from a system by immunizing an animal or by screening an immunoglobulin gene library, and wherein the selected antibody is at least 90%, more preferably at least 95%, even more preferably at least 96%, 97%, 98%, or 99% identical in amino acid sequence to the amino acid sequence encoded by the germline immunoglobulin gene. Typically, an antibody derived from a particular germline sequence will display no more than 10 amino acid differences, more preferably, no more than 5, or even more preferably, no more than 4, 3, 2, or 1 amino acid difference from the amino acid sequence encoded by the germline immunoglobulin gene.

As used herein, the term "heteroantibodies" refers to two or more antibodies, derivatives thereof, or antigen binding regions linked together, at least two of which have different specificities. These different specificities include a binding specificity for an Fc receptor on an effector cell, and a binding specificity for an antigen or epitope on a target cell, e.g., a tumor cell.

The antibodies described herein may be monoclonal antibodies. The term "monoclonal antibody" as used herein refers to a preparation of antibody molecules of single molecular composition. A monoclonal antibody displays a single binding specificity and affinity. In one embodiment, the monoclonal antibodies are produced by a hybridoma which includes a B cell obtained from a non-human animal, e.g., mouse, fused to an immortalized cell.

The antibodies described herein may be recombinant antibodies. The term "recombinant antibody", as used herein, includes all antibodies that are prepared, expressed, created or isolated by recombinant means, such as (a) antibodies isolated from an animal (e.g., a mouse) that is transgenic or transchromosomal with respect to the immunoglobulin genes or a hybridoma prepared therefrom, (b) antibodies isolated from a host cell transformed to express the antibody, e.g., from a transfectoma, (c) antibodies isolated from a recombinant, combinatorial antibody library, and (d) antibodies prepared, expressed, created or isolated by any other means that involve splicing of immunoglobulin gene sequences to other DNA sequences.

The term "transfectoma", as used herein, includes recombinant eukaryotic host cells expressing an antibody, such as CHO cells, NS/0 cells, HEK293 cells, HEK293T cells, plant cells, or fungi, including yeast cells.

As used herein, a "heterologous antibody" is defined in relation to a transgenic organism producing such an antibody. This term refers to an antibody having an amino acid sequence or an encoding nucleic acid sequence corresponding to that found in an organism not consisting of the transgenic organism, and being generally derived from a species other than the transgenic organism.

As used herein, a "heterohybrid antibody" refers to an antibody having light and heavy chains of different organismal origins. For example, an antibody having a human heavy chain associated with a murine light chain is a heterohybrid antibody.

The invention includes all antibodies and derivatives of antibodies as described herein which for the purposes of the invention are encompassed by the term "antibody". The term "antibody derivatives" refers to any modified form of an antibody, e.g., a conjugate of the antibody and another agent or antibody, or an antibody fragment.

The antibodies described herein are preferably isolated. An "isolated antibody" as used herein, is intended to refer to an antibody which is substantially free of other antibodies having different antigenic specificities (e.g., an isolated antibody that specifically binds to PLAC1 is substantially free of antibodies that specifically bind antigens other than PLAC1). An isolated antibody that specifically binds to an epitope, isoform or variant of human PLAC1 may, however, have cross-reactivity to other related antigens, e.g., from other species (e.g., PLAC1 species homologs). Moreover, an isolated antibody may be substantially free of other cellular material and/or chemicals. In one embodiment of the invention, a combination of "isolated" monoclonal antibodies relates to antibodies having different specificities and being combined in a well defined composition or mixture.

The term "binding" according to the invention preferably relates to a specific binding.

According to the present invention, an antibody is capable of binding to a predetermined target if it has a significant affinity for said predetermined target and binds to said predetermined target in standard assays. "Affinity" or "binding affinity" is often measured by equilibrium dissociation constant (K_{D}). Preferably, the term "significant affinity" refers to the binding to a predetermined target with a dissociation constant (K_{D}) of 10⁻⁵ M or lower, 10⁻⁶ M or lower, 10⁻⁷ M or lower, 10⁻⁸ M or lower, 10⁻⁹ M or lower, 10⁻¹⁰ M or lower, 10⁻¹¹ M or lower, or 10⁻¹² M or lower.

An antibody is not (substantially) capable of binding to a target if it has no significant affinity for said target and does not bind significantly, in particular does not bind detectably, to said target in standard assays. Preferably, the antibody does not detectably bind to said target if present in a concentration of up to 2, preferably 10, more preferably 20, in particular 50 or 100 µg/ml or higher. Preferably, an antibody has no significant affinity for a target if it binds to said target with a K_{D} that is at least 10-fold, 100-fold, 10³-fold, 10⁴-fold, 10⁵-fold, or 10⁶-fold higher than the K_{D} for binding to the predetermined target to which the antibody is capable of binding. For example, if the K_{D} for binding of an antibody to the target to which the antibody is capable of binding is 10⁻⁷ M, the K_{D} for binding to a target for which the antibody has no significant affinity would be is at least 10⁻⁶ M, 10⁻⁵ M, 10⁻⁴ M, 10⁻³ M, 10⁻² M, or 10⁻¹ M.

An antibody is specific for a predetermined target if it is capable of binding to said predetermined target while it is not capable of binding to other targets, i.e. has no significant affinity for other targets and does not significantly bind to other targets in standard assays. According to the invention, an antibody is specific for PLAC1 if it is capable of binding to PLAC1 but is not (substantially) capable of binding to other targets. Preferably, an antibody is specific for PLAC1 if the affinity for and the binding to such other targets does not significantly exceed the affinity for or binding to PLAC1-unrelated proteins such as bovine serum albumin (BSA), casein, human serum albumin (HSA) or non-claudin transmembrane proteins such as MHC molecules or transferrin receptor or any other specified polypeptide. Preferably, an antibody is specific for a predetermined target if it binds to said target with a K_{D} that is at least 10-fold, 100-fold, 10³-fold, 10⁴-fold, 10⁵-fold, or 10⁶-fold lower than the K_{D} for binding to a target for which it is not specific. For example, if the K_{D} for binding of an antibody to the target for which it is specific is 10⁻⁷ M, the K_{D} for binding to a target for which it is not specific would be at least 10⁻⁶ M, 10⁻⁵ M, 10⁻⁴ M, 10⁻³ M, 10⁻² M, or 10⁻¹ M.

Binding of an antibody to a target can be determined experimentally using any suitable method; see, for example, Berzofsky et al., "Antibody-Antigen Interactions" In Fundamental Immunology, Paul, W. E., Ed., Raven Press New York, N Y (1984), Kuby, Janis Immunology, W. H. Freeman and Company New York, N Y (1992), and methods described herein. Affinities may be readily determined using conventional techniques, such as by equilibrium dialysis; by using the BIAcore 2000 instrument, using general procedures outlined by the manufacturer; by radioimmunoassay using radiolabeled target antigen; or by another method known to the skilled artisan. The affinity data may be analyzed, for example, by the method of Scatchard et al., Ann N.Y. Acad. ScL, 51:660 (1949). The measured affinity of a particular antibody-antigen interaction can vary if measured under different conditions, e.g., salt concentration, pH. Thus, measurements of affinity and other antigen-binding parameters, e.g., K_{D}, IC₅₀, are preferably made with standardized solutions of antibody and antigen, and a standardized buffer.

As used herein, "isotype" refers to the antibody class (e.g., IgM or IgG1) that is encoded by heavy chain constant region genes.

As used herein, "isotype switching" refers to the phenomenon by which the class, or isotype, of an antibody changes from one Ig class to one of the other Ig classes.

The term "naturally occurring" as used herein as applied to an object refers to the fact that an object can be found in nature. For example, a polypeptide or polynucleotide sequence that is present in an organism (including viruses) that can be isolated from a source in nature and which has not been intentionally modified by man in the laboratory is naturally occurring.

The term "rearranged" as used herein refers to a configuration of a heavy chain or light chain immunoglobulin locus wherein a V segment is positioned immediately adjacent to a D-J or J segment in a conformation encoding essentially a complete VH or VL domain, respectively. A rearranged immunoglobulin (antibody) gene locus can be identified by comparison to germline DNA; a rearranged locus will have at least one recombined heptamer/nonamer homology element.

The term "unrearranged" or "germline configuration" as used herein in reference to a V segment refers to the configuration wherein the V segment is not recombined so as to be immediately adjacent to a D or J segment.

The term "nucleic acid molecule", as used herein, is intended to include DNA molecules and RNA molecules. A nucleic acid molecule may be single-stranded or double-stranded, but preferably is double-stranded DNA.

According to the invention, the term "expression" is used in its most general meaning and comprises the production of RNA or of RNA and protein/peptide. It also comprises partial expression of nucleic acids. Furthermore, expression may be carried out transiently or stably.

For the purposes of the present invention, "variants" of an amino acid sequence comprise amino acid insertion variants, amino acid addition variants, amino acid deletion variants and/or amino acid substitution variants. Amino acid deletion variants that comprise the deletion at the N-terminal and/or C-terminal end of the protein are also called N-terminal and/or C-terminal truncation variants.

Amino acid insertion variants comprise insertions of single or two or more amino acids in a particular amino acid sequence. In the case of amino acid sequence variants having an insertion, one or more amino acid residues are inserted into a particular site in an amino acid sequence, although random insertion with appropriate screening of the resulting product is also possible.

Amino acid addition variants comprise amino- and/or carboxy-terminal fusions of one or more amino acids, such as 1, 2, 3, 5, 10, 20, 30, 50, or more amino acids.

Amino acid deletion variants are characterized by the removal of one or more amino acids from the sequence, such as by removal of 1, 2, 3, 5, 10, 20, 30, 50, or more amino acids. The deletions may be in any position of the protein.

Amino acid substitution variants are characterized by at least one residue in the sequence being removed and another residue being inserted in its place. Preference is given to the modifications being in positions in the amino acid sequence which are not conserved between homologous proteins or peptides and/or to replacing amino acids with other ones having similar properties. Preferably, amino acid changes in protein variants are conservative amino acid changes, i.e., substitutions of similarly charged or uncharged amino acids. A conservative amino acid change involves substitution of one of a family of amino acids which are related in their side chains. Naturally occurring amino acids are generally divided into four families: acidic (aspartate, glutamate), basic (lysine, arginine, histidine), non-polar (alanine, valine, leucine, isoleucine, proline, phenylalanine, methionine, tryptophan), and uncharged polar (glycine, asparagine, glutamine, cysteine, serine, threonine, tyrosine) amino acids. Phenylalanine, tryptophan, and tyrosine are sometimes classified jointly as aromatic amino acids.

Preferably the degree of similarity, preferably identity between a given amino acid sequence and an amino acid sequence which is a variant of said given amino acid sequence will be at least about 60%, 65%, 70%, 80%, 81%, 82%, 83%, 84%, 85%, 86%, 87%, 88%, 89%, 90%, 91%, 92%, 93%, 94%, 95%, 96%, 97%, 98%, or 99%. The degree of similarity or identity is given preferably for an amino acid region which is at least about 10%, at least about 20%, at least about 30%, at least about 40%, at least about 50%, at least about 60%, at least about 70%, at least about 80%, at least about 90% or about 100% of the entire length of the reference amino acid sequence. For example, if the reference amino acid sequence consists of 200 amino acids, the degree of similarity or identity is given preferably for at least about 20, at least about 40, at least about 60, at least about 80, at least about 100, at least about 120, at least about 140, at least about 160, at least about 180, or about 200 amino acids, preferably continuous amino acids. In preferred embodiments, the degree of similarity or identity is given for the entire length of the reference amino acid sequence. The alignment for determining sequence similarity, preferably sequence identity can be done with art known tools, preferably using the best sequence alignment, for example, using Align, using standard settings, preferably EMBOSS::needle, Matrix: Blosum62, Gap Open 10.0, Gap Extend 0.5.

"Sequence similarity" indicates the percentage of amino acids that either are identical or that represent conservative amino acid substitutions. "Sequence identity" between two amino acid sequences indicates the percentage of amino acids that are identical between the sequences.

The term "percentage identity" is intended to denote a percentage of amino acid residues which are identical between the two sequences to be compared, obtained after the best alignment, this percentage being purely statistical and the differences between the two sequences being distributed randomly and over their entire length. Sequence comparisons between two amino acid sequences are conventionally carried out by comparing these sequences after having aligned them optimally, said comparison being carried out by segment or by "window of comparison" in order to identify and compare local regions of sequence similarity. The optimal alignment of the sequences for comparison may be produced, besides manually, by means of the local homology algorithm of Smith and Waterman, 1981, Ads App. Math. 2, 482, by means of the local homology algorithm of Neddleman and Wunsch, 1970, J. Mol. Biol. 48, 443, by means of the similarity search method of Pearson and Lipman, 1988, Proc. Natl Acad. Sci. USA 85, 2444, or by means of computer programs which use these algorithms (GAP, BESTFIT, FASTA, BLAST P, BLAST N and TFASTA in Wisconsin Genetics Software Package, Genetics Computer Group, 575 Science Drive, Madison, Wis.).

The percentage identity is calculated by determining the number of identical positions between the two sequences being compared, dividing this number by the number of positions compared and multiplying the result obtained by 100 so as to obtain the percentage identity between these two sequences.

The terms "transgenic animal" refers to an animal having a genome comprising one or more transgenes, preferably heavy and/or light chain transgenes, or transchromosomes (either integrated or non-integrated into the animal's natural genomic DNA) and which is preferably capable of expressing the transgenes. For example, a transgenic mouse can have a human light chain transgene and either a human heavy chain transgene or human heavy chain transchromosome, such that the mouse produces human anti-PLAC1 antibodies when immunized with PLAC1 antigen and/or cells expressing PLAC1. The human heavy chain transgene can be integrated into the chromosomal DNA of the mouse, as is the case for transgenic mice, e.g., HuMAb mice, such as HCo7 or HCol2 mice, or the human heavy chain transgene can be maintained extrachromosomally, as is the case for transchromosomal (e.g., KM) mice as described in WO 02/43478. Such transgenic and transchromosomal mice may be capable of producing multiple isotypes of human monoclonal antibodies to PLAC1 (e.g., IgG, IgA and/or IgE) by undergoing V-D-J recombination and isotype switching.

"Reduce" or "inhibit" as used herein means the ability to cause an overall decrease, preferably of 5% or greater, 10% or greater, 20% or greater, more preferably of 50% or greater, and most preferably of 75% or greater, in the level, e.g. in the level of proliferation of cells.

Antibodies described herein can be produced by a variety of techniques, including conventional monoclonal antibody methodology, e.g., the standard somatic cell hybridization technique of Kohler and Milstein, Nature 256: 495 (1975). Although somatic cell hybridization procedures are preferred, in principle, other techniques for producing monoclonal antibodies can be employed, e.g., viral or oncogenic transformation of B-lymphocytes or phage display techniques using libraries of antibody genes.

The preferred animal system for preparing hybridomas that secrete monoclonal antibodies is the murine system. Hybridoma production in the mouse is a very well established procedure. Immunization protocols and techniques for isolation of immunized splenocytes for fusion are known in the art. Fusion partners (e.g., murine myeloma cells) and fusion procedures are also known.

Other preferred animal systems for preparing hybridomas that secrete monoclonal antibodies are the rat and the rabbit system (e.g. described in Spieker-Polet et al., Proc. Natl. Acad. Sci. U.S.A. 92:9348 (1995), see also Rossi et al., Am. J. Clin. Pathol. 124: 295 (2005)).

In yet another preferred embodiment, human monoclonal antibodies can be generated using transgenic or transchromosomal mice carrying parts of the human immune system rather than the mouse system. These transgenic and transchromosomic mice include mice known as HuMAb mice and KM mice, respectively, and are collectively referred to herein as "transgenic mice." The production of human antibodies in such transgenic mice can be performed as described in detail for CD20 in WO2004 035607

Yet another strategy for generating monoclonal antibodies is to directly isolate genes encoding antibodies from lymphocytes producing antibodies of defined specificity e.g. see Babcock et al., 1996; A novel strategy for generating monoclonal antibodies from single, isolated lymphocytes producing antibodies of defined specificities. For details of recombinant antibody engineering see also Welschof and Kraus, Recombinant antibodies for cancer therapy ISBN-0-89603-918-8 and Benny K.C. Lo Antibody Engineering ISBN 1-58829-092-1.

To generate antibodies, mice can be immunized with carrier-conjugated peptides derived from the antigen sequence, i.e. the sequence against which the antibodies are to be directed, an enriched preparation of recombinantly expressed antigen or fragments thereof and/or cells expressing the antigen, as described. Alternatively, mice can be immunized with DNA encoding the antigen or fragments thereof. In the event that immunizations using a purified or enriched preparation of the antigen do not result in antibodies, mice can also be immunized with cells expressing the antigen, e.g., a cell line, to promote immune responses.

The immune response can be monitored over the course of the immunization protocol with plasma and serum samples being obtained by tail vein or retroorbital bleeds. Mice with sufficient titers of immunoglobulin can be used for fusions. Mice can be boosted intraperitoneally or intravenously with antigen expressing cells 3 days before sacrifice and removal of the spleen to increase the rate of specific antibody secreting hybridomas.

To generate hybridomas producing monoclonal antibodies, splenocytes and lymph node cells from immunized mice can be isolated and fused to an appropriate immortalized cell line, such as a mouse myeloma cell line. The resulting hybridomas can then be screened for the production of antigen-specific antibodies. Individual wells can then be screened by ELISA for antibody secreting hybridomas. By Immunofluorescence and FACS analysis using antigen expressing cells, antibodies with specificity for the antigen can be identified. The antibody secreting hybridomas can be replated, screened again, and if still positive for monoclonal antibodies can be subcloned by limiting dilution. The stable subclones can then be cultured in vitro to generate antibody in tissue culture medium for characterization.

Antibodies also can be produced in a host cell transfectoma using, for example, a combination of recombinant DNA techniques and gene transfection methods as are well known in the art (Morrison, S. (1985) Science 229: 1202).

For example, in one embodiment, the gene(s) of interest, e.g., antibody genes, can be ligated into an expression vector such as a eukaryotic expression plasmid such as used by the GS gene expression system disclosed in WO 87/04462, WO 89/01036 and EP 338 841 or other expression systems well known in the art. The purified plasmid with the cloned antibody genes can be introduced in eukaryotic host cells such as CHO cells, NS/0 cells, HEK293T cells or HEK293 cells or alternatively other eukaryotic cells like plant derived cells, fungal or yeast cells. The method used to introduce these genes can be methods described in the art such as electroporation, lipofectine, lipofectamine or others. After introduction of these antibody genes in the host cells, cells expressing the antibody can be identified and selected. These cells represent the transfectomas which can then be amplified for their expression level and upscaled to produce antibodies. Recombinant antibodies can be isolated and purified from these culture supernatants and/or cells.

Alternatively, the cloned antibody genes can be expressed in other expression systems, including prokaryotic cells, such as microorganisms, e.g. E. coli. Furthermore, the antibodies can be produced in transgenic non-human animals, such as in milk from sheep and rabbits or in eggs from hens, or in transgenic plants; see e.g. Verma, R., et al. (1998) J. Immunol. Meth. 216: 165-181; Pollock, et al. (1999) J. Immunol. Meth. 231: 147-157; and Fischer, R., et al. (1999) Biol. Chem. 380: 825-839.

### Chimerization

Murine monoclonal antibodies can be used as therapeutic antibodies in humans when labeled with toxins or radioactive isotopes. Nonlabeled murine antibodies are highly immunogenic in man when repetitively applied leading to reduction of the therapeutic effect. The main immunogenicity is mediated by the heavy chain constant regions. The immunogenicity of murine antibodies in man can be reduced or completely avoided if respective antibodies are chimerized or humanized. Chimeric antibodies are antibodies, the different portions of which are derived from different animal species, such as those having a variable region derived from a murine antibody and a human immunoglobulin constant region. Chimerisation of antibodies is achieved by joining of the variable regions of the murine antibody heavy and light chain with the constant region of human heavy and light chain (e.g. as described by Kraus et al., in Methods in Molecular Biology series, Recombinant antibodies for cancer therapy ISBN-0-89603-918-8). In a preferred embodiment chimeric antibodies are generated by joining human kappa-light chain constant region to murine light chain variable region. In an also preferred embodiment chimeric antibodies can be generated by joining human lambda-light chain constant region to murine light chain variable region. The preferred heavy chain constant regions for generation of chimeric antibodies are IgG1, IgG3 and IgG4. Other preferred heavy chain constant regions for generation of chimeric antibodies are IgG2, IgA, IgD and IgM.

### Humanization

Antibodies interact with target antigens predominantly through amino acid residues that are located in the six heavy and light chain complementarity determining regions (CDRs). For this reason, the amino acid sequences within CDRs are more diverse between individual antibodies than sequences outside of CDRs. Because CDR sequences are responsible for most antibody-antigen interactions, it is possible to express recombinant antibodies that mimic the properties of specific naturally occurring antibodies by constructing expression vectors that include CDR sequences from the specific naturally occurring antibody grafted onto framework sequences from a different antibody with different properties (see, e.g., Riechmann, L. et al. (1998) Nature 332: 323-327; Jones, P. et al. (1986) Nature 321: 522-525; and Queen, C. et al. (1989) Proc. Natl. Acad. Sci. U. S. A. 86: 10029-10033). Such framework sequences can be obtained from public DNA databases that include germline antibody gene sequences. These germline sequences will differ from mature antibody gene sequences because they will not include completely assembled variable genes, which are formed by V (D) J joining during B cell maturation. Germline gene sequences will also differ from the sequences of a high affinity secondary repertoire antibody at individual evenly across the variable region.

The ability of antibodies to bind an antigen can be determined using standard binding assays (e.g., ELISA, Western Blot, Immunofluorescence and flow cytometric analysis).

To purify antibodies, selected hybridomas can be grown in two-liter spinner-flasks for monoclonal antibody purification. Alternatively, antibodies can be produced in dialysis based bioreactors. Supernatants can be filtered and, if necessary, concentrated before affinity chromatography with protein G-sepharose or protein A-sepharose. Eluted IgG can be checked by gel electrophoresis and high performance liquid chromatography to ensure purity. The buffer solution can be exchanged into PBS, and the concentration can be determined by OD280 using 1.43 extinction coefficient. The monoclonal antibodies can be aliquoted and stored at -80°C.

To determine if the selected monoclonal antibodies bind to unique epitopes, site-directed or multi-site directed mutagenesis can be used.

To determine the isotype of antibodies, isotype ELISAs with various commercial kits (e.g. Zymed, Roche Diagnostics) can be performed. Wells of microtiter plates can be coated with anti-mouse Ig. After blocking, the plates are reacted with monoclonal antibodies or purified isotype controls, at ambient temperature for two hours. The wells can then be reacted with either mouse IgG1, IgG2a, IgG2b or IgG3, IgA or mouse IgM-specific peroxidase-conjugated probes. After washing, the plates can be developed with ABTS substrate (1 mg/ml) and analyzed at OD of 405-650. Alternatively, the IsoStrip Mouse Monoclonal Antibody Isotyping Kit (Roche, Cat. No. 1493027) may be used as described by the manufacturer.

In order to demonstrate presence of antibodies in sera of immunized mice or binding of monoclonal antibodies to living cells expressing antigen, flow cytometry can be used. Cell lines expressing naturally or after transfection antigen and negative controls lacking antigen expression (grown under standard growth conditions) can be mixed with various concentrations of monoclonal antibodies in hybridoma supernatants or in PBS containing 1% FBS, and can be incubated at 4°C for 30 min. After washing, the APC- or Alexa647-labeled anti IgG antibody can bind to antigen-bound monoclonal antibody under the same conditions as the primary antibody staining. The samples can be analyzed by flow cytometry with a FACS instrument using light and side scatter properties to gate on single, living cells. In order to distinguish antigen-specific monoclonal antibodies from non-specific binders in a single measurement, the method of co-transfection can be employed. Cells transiently transfected with plasmids encoding antigen and a fluorescent marker can be stained as described above. Transfected cells can be detected in a different fluorescence channel than antibody-stained cells. As the majority of transfected cells express both transgenes, antigen-specific monoclonal antibodies bind preferentially to fluorescence marker expressing cells, whereas non-specific antibodies bind in a comparable ratio to non-transfected cells. An alternative assay using fluorescence microscopy may be used in addition to or instead of the flow cytometry assay. Cells can be stained exactly as described above and examined by fluorescence microscopy.

In order to demonstrate presence of antibodies in sera of immunized mice or binding of monoclonal antibodies to living cells expressing antigen, immunofluorescence microscopy analysis can be used. For example, cell lines expressing either spontaneously or after transfection antigen and negative controls lacking antigen expression are grown in chamber slides under standard growth conditions in DMEM/F12 medium, supplemented with 10 % fetal calf serum (FCS), 2 mM L-glutamine, 100 IU/ml penicillin and 100 µg/ml streptomycin. Cells can then be fixed with methanol or paraformaldehyde or left untreated. Cells can then be reacted with monoclonal antibodies against the antigen for 30 min. at 25°C. After washing, cells can be reacted with an Alexa555-labelled anti-mouse IgG secondary antibody (Molecular Probes) under the same conditions. Cells can then be examined by fluorescence microscopy.

Cell extracts from cells expressing antigen and appropriate negative controls can be prepared and subjected to sodium dodecyl sulfate (SDS) polyacrylamide gel electrophoresis. After electrophoresis, the separated antigens will be transferred to nitrocellulose membranes, blocked, and probed with the monoclonal antibodies to be tested. IgG binding can be detected using anti-mouse IgG peroxidase and developed with ECL substrate.

Antibodies can be further tested for reactivity with antigen by Immunohistochemistry in a manner well known to the skilled person, e.g. using paraformaldehyde or acetone fixed cryosections or paraffin embedded tissue sections fixed with paraformaldehyde from non-cancer tissue or cancer tissue samples obtained from patients during routine surgical procedures or from mice carrying xenografted tumors inoculated with cell lines expressing spontaneously or after transfection antigen. For immunostaining, antibodies reactive to antigen can be incubated followed by horseradish-peroxidase conjugated goat anti-mouse or goat anti-rabbit antibodies (DAKO) according to the vendor's instructions.

A general inhibition of cell proliferation by antibodies can be detected with commercially available kits. The DELFIA Cell Proliferation Kit (Perkin-Elmer, Cat. No. AD0200) is a non-isotopic immunoassay based on the measurement of 5-bromo-2'-deoxyuridine (BrdU) incorporation during DNA synthesis of proliferating cells in microplates. Incorporated BrdU is detected using europium labelled monoclonal antibody. To allow antibody detection, cells are fixed and DNA denatured using Fix solution. Unbound antibody is washed away and DELFIA inducer is added to dissociate europium ions from the labelled antibody into solution, where they form highly fluorescent chelates with components of the DELFIA Inducer. The fluorescence measured - utilizing time-resolved fluorometry in the detection - is proportional to the DNA synthesis in the cell of each well.

Mapping of epitopes recognized by antibodies can be performed as described in detail in "Epitope Mapping Protocols (Methods in Molecular Biology) by Glenn E. Morris ISBN-089603-375-9 and in "Epitope Mapping: A Practical Approach" Practical Approach Series, 248 by Olwyn M. R. Westwood, Frank C. Hay.

Pharmaceutical compositions are usually provided in a uniform dosage form and may be prepared in a manner known per se. The pharmaceutical composition of the invention may e.g. be in the form of a solution or suspension.

The pharmaceutical composition of the invention may comprise salts, buffer substances, preservatives, carriers, diluents and/or excipients all of which are preferably pharmaceutically acceptable. The term "pharmaceutically acceptable" refers to the non-toxicity of a material which does not interact with the action of the active component of the pharmaceutical composition.

Salts which are not pharmaceutically acceptable may be used for preparing pharmaceutically acceptable salts and are included in the invention. Pharmaceutically acceptable salts of this kind comprise in a non limiting way those prepared from the following acids: hydrochloric, hydrobromic, sulfuric, nitric, phosphoric, maleic, acetic, salicylic, citric, formic, malonic, succinic acids, and the like. Pharmaceutically acceptable salts may also be prepared as alkali metal salts or alkaline earth metal salts, such as sodium salts, potassium salts or calcium salts.

Suitable buffer substances for use in the pharmaceutical composition of the invention include acetic acid in a salt, citric acid in a salt, boric acid in a salt and phosphoric acid in a salt.

Suitable preservatives for use in the pharmaceutical composition of the invention include benzalkonium chloride, chlorobutanol, paraben and thimerosal.

An injectable formulation may comprise a pharmaceutically acceptable excipient such as Ringer Lactate.

The term "carrier" refers to an organic or inorganic component, of a natural or synthetic nature, in which the active component is combined in order to facilitate, enhance or enable application. According to the invention, the term "carrier" also includes one or more compatible solid or liquid fillers, diluents or encapsulating substances, which are suitable for administration to a patient.

Possible carrier substances for parenteral administration are e.g. sterile water, Ringer, Ringer lactate, sterile sodium chloride solution, polyalkylene glycols, hydrogenated naphthalenes and, in particular, biocompatible lactide polymers, lactide/glycolide copolymers or polyoxyethylene/polyoxy- propylene copolymers.

The term "excipient" when used herein is intended to indicate all substances which may be present in a pharmaceutical composition of the present invention and which are not active ingredients such as, e.g., carriers, binders, lubricants, thickeners, surface active agents, preservatives, emulsifiers, buffers, flavoring agents, or colorants.

The agents and compositions described herein may be administered via any conventional route, such as by parenteral administration including by injection or infusion. Administration is preferably parenterally, e.g. intravenously, intraarterially, subcutaneously, intradermally or intramuscularly.

Compositions suitable for parenteral administration usually comprise a sterile aqueous or nonaqueous preparation of the active compound, which is preferably isotonic to the blood of the recipient. Examples of compatible carriers and solvents are Ringer solution and isotonic sodium chloride solution. In addition, usually sterile, fixed oils are used as solution or suspension medium.

The agents and compositions described herein are administered in effective amounts. An "effective amount" refers to the amount which achieves a desired reaction or a desired effect alone or together with further doses. In the case of treatment of a particular disease or of a particular condition, the desired reaction preferably relates to inhibition of the course of the disease. This comprises slowing down the progress of the disease and, in particular, interrupting or reversing the progress of the disease. The desired reaction in a treatment of a disease or of a condition may also be delay of the onset or a prevention of the onset of said disease or said condition.

An effective amount of an agent or composition described herein will depend on the condition to be treated, the severeness of the disease, the individual parameters of the patient, including age, physiological condition, size and weight, the duration of treatment, the type of an accompanying therapy (if present), the specific route of administration and similar factors. Accordingly, the doses administered of the agents described herein may depend on various of such parameters. In the case that a reaction in a patient is insufficient with an initial dose, higher doses (or effectively higher doses achieved by a different, more localized route of administration) may be used.

The agents and compositions described herein can be administered to patients, e.g., in vivo, to treat or prevent a variety of disorders such as those described herein. Preferred patients include human patients having disorders that can be corrected or ameliorated by administering the agents and compositions described herein. This includes disorders involving cells characterized by an altered expression pattern of PLAC1 and/or an altered pattern of association of PLAC1 with their cell surface compared to normal cells.

For example, in one embodiment, antibodies described herein can be used to treat a patient with a cancer disease, e.g., a cancer disease characterized by the presence of cancer cells expressing PLAC1 and/or being characterized by association of PLAC1 with their cell surface. Examples of cancer diseases which can be treated and/or prevented encompass all PLAC1 expressing cancers and include breast cancer, lung cancer, gastric cancer, ovarian cancer, hepatocellular cancer, colon cancer, pancreatic cancer, esophageal cancer, head & neck cancer, kidney cancer, in particular renal cell carcinoma, prostate cancer, liver cancer, melanoma, sarcoma, myeloma, neuroblastoma, placental choriocarcinoma, cervical cancer, and thyroid cancer, and the metastatic forms thereof. These cancers may be in early, intermediate or advanced stages, e.g. metastasis. In one embodiment, the cancer disease is metastatic cancer in the lung.

The pharmaceutical compositions and methods of treatment described according to the invention may also be used for immunization or vaccination to prevent a disease described herein.

As described above, antibodies described herein can be co-administered with one or more other therapeutic agents, e.g., a cytotoxic agent, a radiotoxic agent, antiangiogenic agent or and immunosuppressive agent to reduce the induction of immune responses against the antibodies. The antibody can be linked to the agent (as an immunocomplex) or can be administered separate from the agent. In the latter case (separate administration), the antibody can be administered before, after or concurrently with the agent or can be co-administered with other known therapies, e.g., an anti-cancer therapy, e.g., radiation. Such therapeutic agents include, among others, anti-neoplastic agents such as listed above. Co-administration of the antibodies with chemotherapeutic agents may provide two anti-cancer agents which operate via different mechanisms yielding a cytotoxic effect to cancer cells. Such co-administration can solve problems due to development of resistance to drugs or a change in the antigenicity of the cancer cells which would render them unreactive with the antibody.

The present invention is further illustrated by the following examples.

### EXAMPLES

The techniques and methods mentioned herein are carried out in a manner known per se and as described, for example, in Sambrook et al., Molecular Cloning: A Laboratory Manual, 2nd Edition (1989) Cold Spring Harbor Laboratory Press, Cold Spring Harbor, N.Y, or as described below. All methods including the use of kits and reagents are carried out according to the manufacturers' information.

### Example 1: Precipitation properties of different PLAC1 specific antibody mixtures

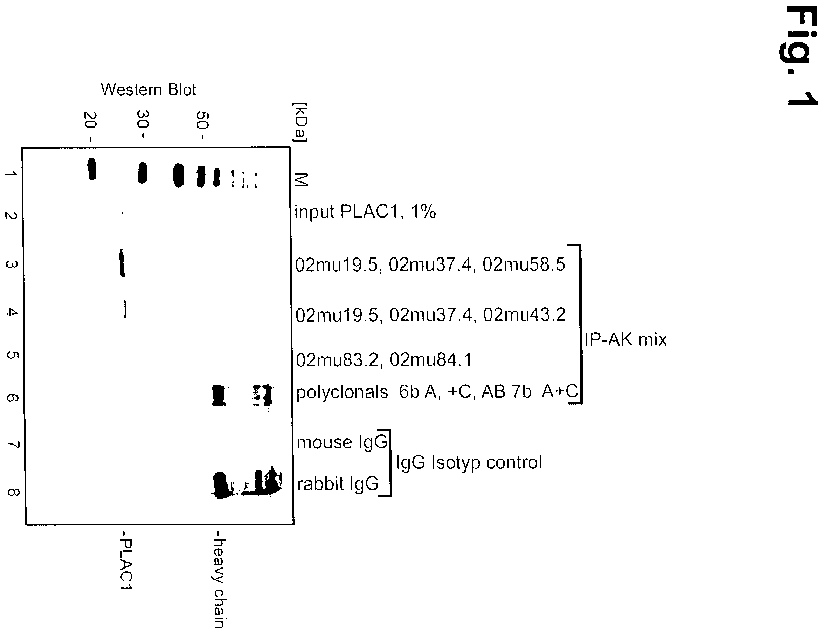

Human PLAC1 was immunoprecipitated by different antibody mixtures from BeWo cell lysate; cf. Figure 1. Lane 1 shows 1% of BeWo cell extract used, lane 2-4 show mixtures of monoclonal antibodies, recognizing the C-terminal (lanes 3, 4, 1:1:1) or N-terminal (lane 5, 1:1) part of human PLAC1. Lane 6 shows precipitation properties of a mixture of polyclonal antibodies (1:1), directed against the C-terminal part of human PLAC1. Lanes 7 and 8 show mouse or rabbit IgG control immunoprecipitations from BeWo lysate, respectively.

This demonstrates that a mixture of PLAC1 antibodies can much better precipitate native PLAC1 than other mixtures. This effect is probably due to higher coverage of accessible epitopes in multimeric PLAC1.

### Example 2: Human PLAC1 specific monoclonal ABs block the binding of PLAC1 to FGF7

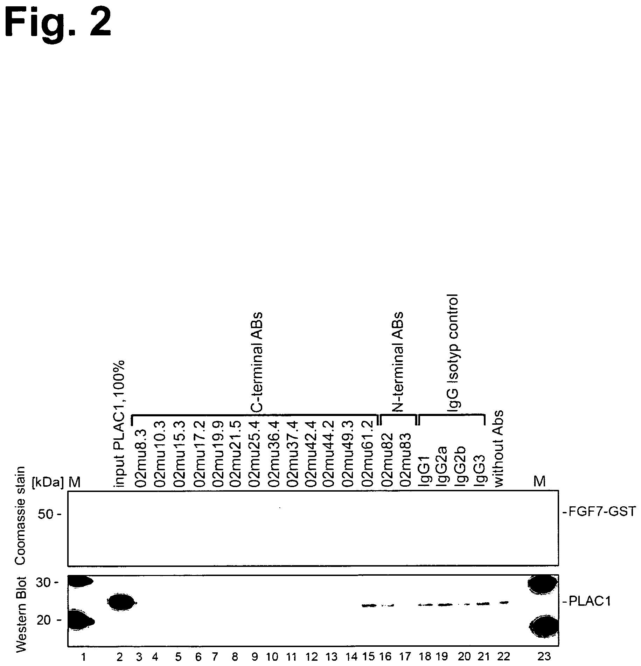

Bacterially expressed and purified GST-FGF7 was immobilized to a sepharose matrix (1mg each, lanes 3-22, total input of used GST-FGF7 protein, gel after blotting, upper panel of Figure 2) and incubated with lysate from PLAC1 overexpressing HEK293T cells that was preincubated for one hour at 4°C with different monoclonal antibodies (10mg), directed against human PLAC1. Lane 2 shows 100% PLAC1 lysate used in this assay. Lanes 3-14 show PLAC1 AB directed against the C-terminal part of PLAC1, lanes 15-16 AB directed against the N-terminal part of PLAC1. Lanes 18-21 show IgG isotype controls and lane 22 shows binding of PLAC1 to GST-FGF7 without preincubation with antibodies. Interactions were assessed by elution of the interacting proteins by boiling in SDS-sample buffer followed by SDS-PAGE and immunoblotting with specific antibodies (lower panel of Figure 2).

This demonstrates that specific monoclonal antibodies directed against human PLAC1 can block the interaction between rec. GST-FGF7 and PLAC1. Interestingly all the tested antibodies, derived from a condensed list reflecting their Western blotting and IP properties can block the binding. Only antibodies that can not immunoprecipitate (lane 15, 16) PLAC1 are not effecting FGF7 binding in this *in vitro* assay.

### Example 3: FGFR2IIIb and PLAC1 co-immunprecipitate with FGF7

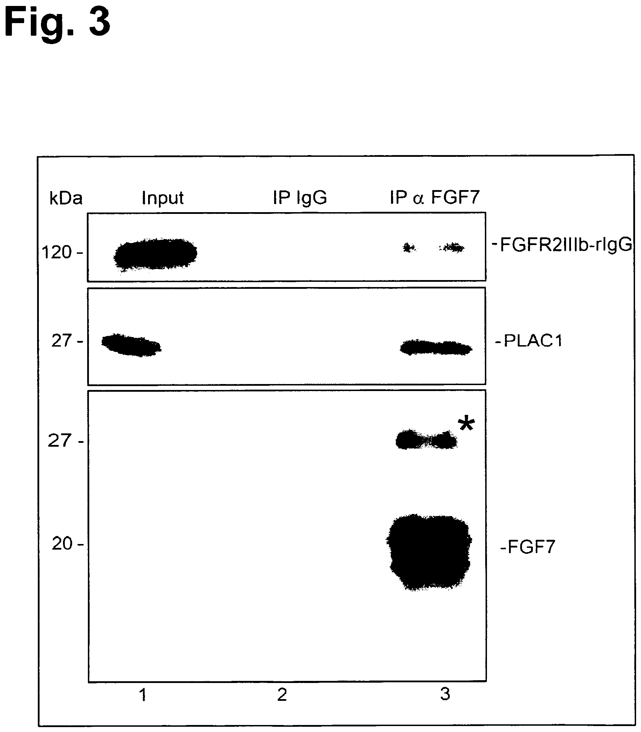

HEK293T cells expressing FGF7, PLAC1 and a rabbit-IgG-FGFR2IIIb (D1-D3) fusion protein were subjected to co-immunoprecipitation with an FGF7-specific antibody (lane 3 of Figure 3). IP with IgGs served as control (lane 2 of Figure 3).

This demonstrates that PLAC1 interacts specifically with FGF7 and thereby forms a complex with the FGFR2IIIb receptor in vivo.

### Example 4: PLAC1 activates Akt phosphorylation by specific stimulation and activation of FGFR2IIIb receptor

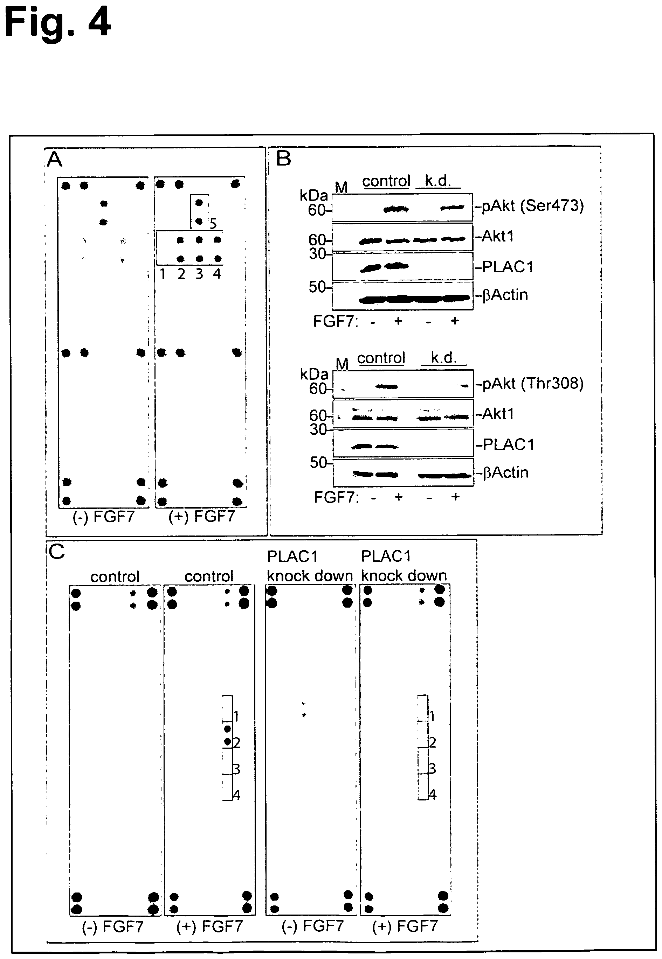

As demonstrated in Figure 4 (A), FGF7 stimulation of BeWo cells leads to a specific phosphorylation of the signal node proteins Akt (on Thr308 (1) and Ser473 (2)) as well as MAPK (3). Also S6 (4) and Src (5) protein is activated. As demonstrated in Figure 4 (B), the stimulation of Akt is influenced by PLAC1. The FGF7 induced phosphorylation of Akt Ser473 (upper panel) is reduced in PLAC1 knock down cells (k.d.) compared to control cells (control). The same effect is detectable for Akt Thr308 (lower panel, k.d. vs. control). For internal control, Akt1, PLAC1 and βAktin was detected with specific antibodies. As demonstrated in Figure 4 (C), only the FGFR2 receptor is activated by FGF7 (2) in contrast to FGFR1 (1), FGFR (3) and FGFR (4) (control, left side). In PLAC1 knock down cells (k.d., right side) this FGF7-specific FGFR2 activation is blocked.

### Example 5: FGF7 dependent stimulation of Akt is influenced by PLAC1 in MCF7 cells

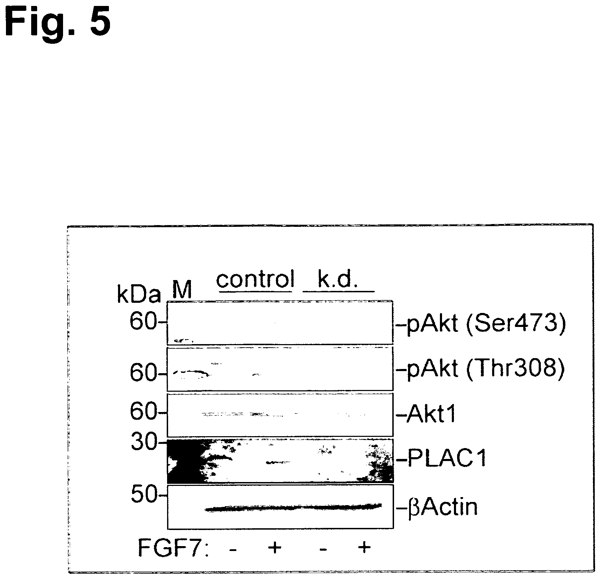

As demonstrated in Figure 5, FGF7 induced phosphorylation of Akt Ser473 (upper panel) is reduced in PLAC1 knock down cells (k.d.) compared to control cells (control). The same effect is detectable for Akt Thr308 (lower panel, k.d. vs. control). For internal control, aktl, PLAC1 and βAktin was detected with specific antibodies.

### Example 6: PLAC1 expression promotes FGF7 mediated proliferation of the breast cancer cell line MCF7

MCF7 cells were transfected with a mixture of two different siRNA duplexes (Qiagen) targeting PLAC1 and a non-silencing control RNA duplex. Cells were cultured at low FCS concentrations supplementing the media with or without FGF7 (0 and 10 ng/ml). Proliferation was measured after 24, 48 and 72 h using the DELFIA cell proliferation kit.

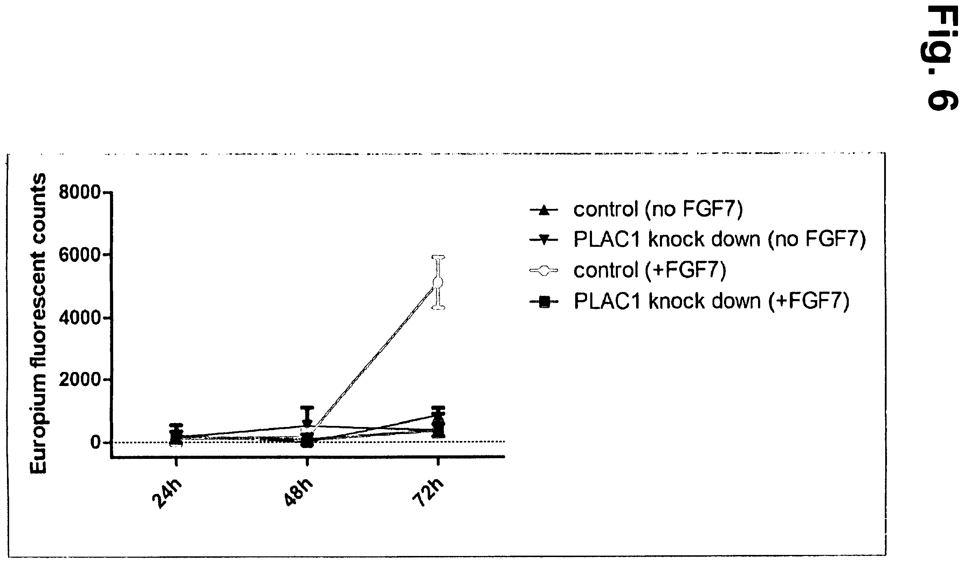

As demonstrated in Figure 6, the presence of PLAC1 is required for the FGF7-mediated proliferation in the cancer cell line MCF7 (breast cancer).

### Example 7: PLAC1 expression promotes FGF7 mediated proliferation of BeWo cells

BeWo cells with a lentiviral knockdown of PLAC1 and respective controls were stimulated with 50 ng/ml FGF7 for 48h. The cell number was determined by crystalviolet staining followed by absorption measurement at 560 nm. Results were normalized to control condition and analysed by ANOVA.

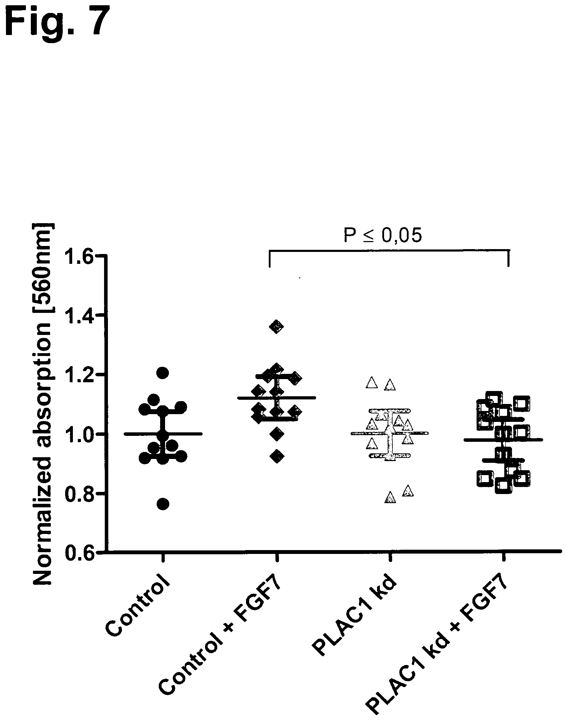

As demonstrated in Figure 7, the presence of PLAC1 is required for the FGF7-mediated proliferation in the cancer cell line BeWo (choriocarcinoma).

### Example 8: hPLAC1 promotes attachment of BeWo cells to cell culture surfaces

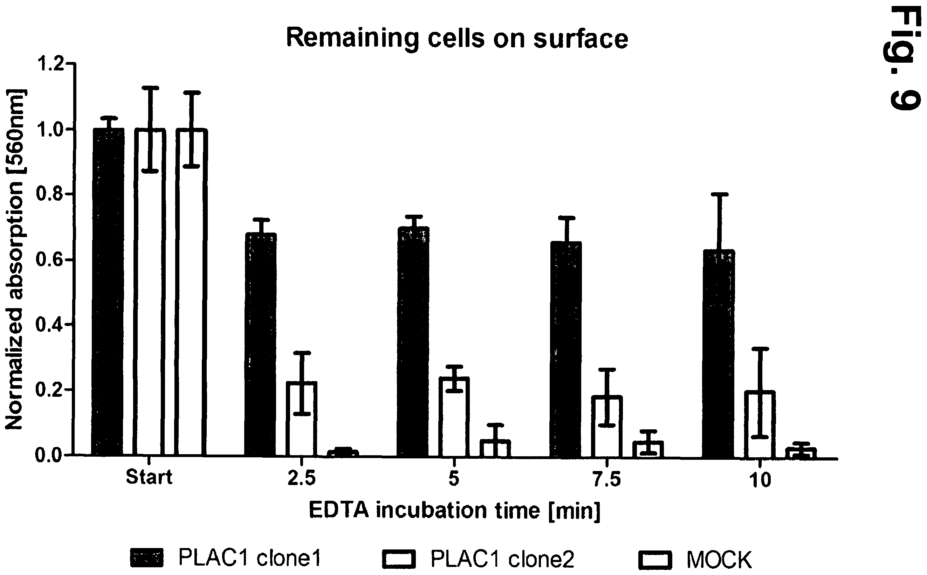

The hPLAC1 dependent detachment was assessed by trypsinization of control or lentiviral hPLAC1 silenced BeWo cells; cf. Figure 9. Remaining cells on surface after trypsinization were determined by crystalviolet staining (left panel) and by counting the number of detached cells after different time points (right panel). The time point at 35 min serves as control for a complete detachment (mean ± SD, N = 4).

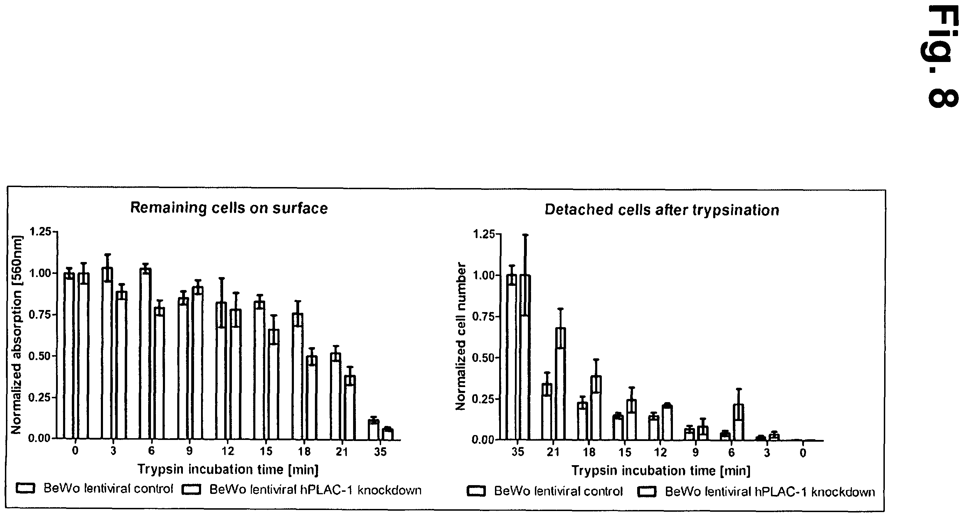

Figure 8 demonstrates that the presence of PLAC1 is required for the attachment of BeWo cells to surfaces.

### Example 9: Detachment assay of PLAC1 stable cell lines

HEK 293 cells stably transfected with PLAC1 (two clones) or corresponding mock control were incubated with 5 mM EDTA to detach cells from surface. Incubation was stopped after defined time points. Remaining cells on surface after EDTA incubation were determined by crystalviolet staining. Photometric quantification was carried out at 560 nm. Data was normalized to the signal of untreated HEK 293 cells (mean ± SD, N = 3).

Figure 9 demonstrates that overexpressed PLAC1 is required for the attachment of HEK cells to surfaces. Thus, PLAC1 reveals to be a general factor for cellular interactions with the surrounding tissue, influencing processes like migration and adhesion.

## Claims

1. A pharmaceutical composition comprising a monoclonal antibody or a mixture of monoclonal antibodies which binds to the C-terminus of PLAC1, preferably to the amino acid sequence spanning from amino acids 23 to 212 of PLAC1 and blocks the interaction between PLAC1 and FGF7 for use in a method for treating cancer or for use in treating cancer metastasis, wherein PLAC1 has the amino acid sequence of SEQ ID NO: 1.

2. The pharmaceutical composition for use according to claim 1 wherein the cancer cells express PLAC1.

3. A monoclonal antibody or a mixture of monoclonal antibodies which binds to the C-terminus of PLAC1, preferably to the amino acid sequence spanning from amino acids 23 to 212 of PLAC1 and blocks the interaction between PLAC1 and FGF7 for use in a method for treating cancer or for use in a method for treating cancer metastasis, wherein PLAC1 has the amino acid sequence of SEQ ID NO: 1.

4. The monoclonal antibody or mixture of monoclonal antibodies for use according to claim 3 wherein the cancer cells express PLAC1.

5. The monoclonal antibody or mixture of monoclonal antibodies for use according to claim 3 or 4 which is capable of immunoprecipitating PLAC1.

6. The monoclonal antibody or mixture of monoclonal antibodies for use according to any one of claims 3 to 5, wherein said mixture of monoclonal antibodies comprises two or more monoclonal antibodies each of said two or more monoclonal antibodies binding to different epitopes of PLAC1.

7. The monoclonal antibody or mixture of monoclonal antibodies for use according to any one of claims 3 to 6, wherein said mixture of monoclonal antibodies comprises one or more monoclonal antibodies binding to PLAC1 and (i) one or more monoclonal antibodies binding to FGF7 and/or (ii) one or more monoclonal antibodies binding to FGFR2IIIb.

8. The monoclonal antibody or mixture of monoclonal antibodies for use according to any one of claims 3 to 7 which inhibits FGF7-dependent phosphorylation of FGFR2IIIb and/or FGF7/FGFR2IIIb-dependent phosphorylation of Akt.

9. The monoclonal antibody or mixture of monoclonal antibodies for use according to claim 8, wherein said phosphorylation of Akt takes place at Thr308 and/or Ser473.

10. The monoclonal antibody or mixture of monoclonal antibodies for use according to any one of claims 3 to 9 which inhibits proliferation, migration and/or attachment of cancer cells.

11. The monoclonal antibody or mixture of monoclonal antibodies for use according to claim 10, wherein said proliferation, migration and/or attachment of cancer cells is FGF7/FGFR2IIIb-dependent.

## Patentansprüche

1. Pharmazeutische Zusammensetzung, die einen monoklonalen Antikörper oder ein Gemisch von monoklonalen Antikörpern umfasst, der/das an den C-Terminus von PLAC1, vorzugsweise an die Aminosäuresequenz, die sich über die Aminosäuren 23 bis 212 von PLAC1 erstreckt, bindet und die Interaktion zwischen PLAC1 und FGF7 blockiert, zur Verwendung in einem Verfahren zur Behandlung von Krebs oder zur Verwendung bei der Behandlung von Krebsmetastasen, wobei PLAC1 die Aminosäuresequenz von SEQ ID NO:1 aufweist.

2. Pharmazeutische Zusammensetzung zur Verwendung gemäß Anspruch 1, wobei die Krebszellen PLAC1 exprimieren.

3. Monoklonaler Antikörper oder Gemisch von monoklonalen Antikörpern, der/das an den C-Terminus von PLAC1, vorzugsweise an die Aminosäuresequenz, die sich über die Aminosäuren 23 bis 212 von PLAC1 erstreckt, bindet und die Interaktion zwischen PLAC1 und FGF7 blockiert, zur Verwendung in einem Verfahren zur Behandlung von Krebs oder zur Verwendung in einem Verfahren zur Behandlung von Krebsmetastasen, wobei PLAC1 die Aminosäuresequenz von SEQ ID NO:1 aufweist.

4. Monoklonaler Antikörper oder Gemisch von monoklonalen Antikörpern zur Verwendung gemäß Anspruch 3, wobei die Krebszellen PLAC1 exprimieren.

5. Monoklonaler Antikörper oder Gemisch von monoklonalen Antikörpern zur Verwendung gemäß Anspruch 3 oder 4, der/das PLAC1 immunpräzipitieren kann.

6. Monoklonaler Antikörper oder Gemisch von monoklonalen Antikörpern zur Verwendung gemäß einem der Ansprüche 3 bis 5, wobei das Gemisch von monoklonalen Antikörpern zwei oder mehrere monoklonale Antikörper umfasst, wobei jeder der zwei oder mehreren monoklonalen Antikörper an verschiedene Epitope von PLAC1 bindet.

7. Monoklonaler Antikörper oder Gemisch von monoklonalen Antikörpern zur Verwendung gemäß einem der Ansprüche 3 bis 6, wobei das Gemisch von monoklonalen Antikörpern einen oder mehrere monoklonale Antikörper, der/die an PLAC1 bindet/binden, und (i) einen oder mehrere monoklonale Antikörper, der/die an FGF7 bindet/binden, und/oder (ii) einen oder mehrere monoklonale Antikörper, der/die an FGFR2IIIb bindet/binden, umfasst.

8. Monoklonaler Antikörper oder Gemisch von monoklonalen Antikörpern zur Verwendung gemäß einem der Ansprüche 3 bis 7, der/das FGF7-abhängige Phosphorylierung von FGFR2IIIb und/oder FGF7/FGFR2IIIb-abhängige Phosphorylierung von Akt hemmt.

9. Monoklonaler Antikörper oder Gemisch von monoklonalen Antikörpern zur Verwendung gemäß Anspruch 8, wobei die Phosphorylierung von Akt an Thr308 und/oder Ser473 stattfindet.

10. Monoklonaler Antikörper oder Gemisch von monoklonalen Antikörpern zur Verwendung gemäß einem der Ansprüche 3 bis 9, der/das Proliferation, Migration und/oder Anlagerung von Krebszellen hemmt.

11. Monoklonaler Antikörper oder Gemisch von monoklonalen Antikörpern zur Verwendung gemäß Anspruch 10, wobei die Proliferation, Migration und/oder Anlagerung von Krebszellen FGF7/FGFR2IIIb-abhängig ist.

## Revendications

1. Composition pharmaceutique comprenant un anticorps monoclonal, ou un mélange d'anticorps monoclonaux, qui se lie à l'extrémité C-terminale de PLAC1, de préférence à la séquence d'acides aminés englobant les acides aminés 23 à 212 de PLAC1, et bloque l'interaction entre PLAC1 et le FGF7, destinée à être utilisée dans un procédé de traitement du cancer ou destinée à être utilisée dans le traitement des métastases du cancer, où PLAC1 possède la séquence d'acides aminés de SEQ ID NO: 1.

2. Composition pharmaceutique destinée à une utilisation selon la revendication 1, où les cellules cancéreuses expriment PLAC1.

3. Anticorps monoclonal, ou mélange d'anticorps monoclonaux, qui se lie à l'extrémité C-terminale de PLAC1, de préférence à la séquence d'acides aminés englobant les acides aminés 23 à 212 de PLAC1, et bloque l'interaction entre PLAC1 et le FGF7, destiné à une utilisation dans un procédé de traitement du cancer ou destiné à une utilisation dans le traitement des métastases du cancer, où PLAC1 possède la séquence d'acides aminés de SEQ ID NO: 1.

4. Anticorps monoclonal, ou mélange d'anticorps monoclonaux, destiné à une utilisation selon la revendication 3, où les cellules cancéreuses expriment PLAC1.

5. Anticorps monoclonal, ou mélange d'anticorps monoclonaux, destiné à une utilisation selon la revendication 3 ou 4, qui est capable d'entraîner une immunoprécipitation de PLAC1.

6. Anticorps monoclonal, ou mélange d'anticorps monoclonaux, destiné à une utilisation selon l'une quelconque des revendications 3 à 5, où ledit mélange d'anticorps monoclonaux comprend deux anticorps monoclonaux ou plus, chacun desdits deux anticorps monoclonaux ou plus se liant à différents épitopes de PLAC1.

7. Anticorps monoclonal, ou mélange d'anticorps monoclonaux, destiné à une utilisation selon l'une quelconque des revendications 3 à 6, où ledit mélange d'anticorps monoclonaux comprend un ou plusieurs anticorps monoclonaux se liant à PLAC1 et (i) un ou plusieurs anticorps monoclonaux se liant au FGF7 et/ou (ii) un ou plusieurs anticorps monoclonaux se liant au FGFR2IIIb.

8. Anticorps monoclonal, ou mélange d'anticorps monoclonaux, destiné à une utilisation selon l'une quelconque des revendications 3 à 7, qui inhibe la phosphorylation du FGFR2IIIb dépendante du FGF7 et/ou la phosphorylation d'Akt dépendante du FGF7/FGFR2IIIb.

9. Anticorps monoclonal, ou mélange d'anticorps monoclonaux, destiné à une utilisation selon la revendication 8, où ladite phosphorylation d'Akt se produit à Thr308 et/ou Ser473.

10. Anticorps monoclonal, ou mélange d'anticorps monoclonaux, destiné à une utilisation selon l'une quelconque des revendications 3 à 9, qui inhibe la prolifération, la migration et/ou l'attachement des cellules cancéreuses.

11. Anticorps monoclonal, ou mélange d'anticorps monoclonaux, destiné à une utilisation selon la revendication 10, où ladite prolifération, ladite migration et/ou ledit attachement des cellules cancéreuses est dépendant(e) du FGF7/FGFR2IIIb.
